# EUROPEAN PATENT APPLICATION

(11) **EP 1 757 703 A2**
(43) Date of publication of application: **28.02.2007**
(21) Application number: 06119359.5
(22) Date of filing: 23.08.2006
(51) Int. Cl.: C12N 15/867, A61K 48/00, C12N 7/04

(54) **Self-inactivating retroviral vector**

(30) Priority: 24.08.2005 EP 05107785; 10.05.2006 EP 06113780
(71) Applicant: Medizinische Hochschule Hannover, 30625 Hannover (DE)
(72) Inventor: Baum, Christopher, Prof. Dr., 22589 Hamburg (DE); Schambach, Axel, Dr., 21220 Seevetal (DE); Bohne, Jens, Dr., 30169 Hannover (DE)
(74) Representative: Taruttis, Stefan Georg

(57) **Abstract**

The invention relates to retroviral vectors, especially to self-inactivating (SIN) gammaretroviral or lentiviral vectors, suitable for producing viral particles at high titers, which can be used for efficient gene transfer into mammalian cells, organs or organisms, e.g. for gene therapy. More specifically, the present invention provides modified 5'-promoter elements in the U3-region of the 5'-LTR of the vector plasmid and 3'-SIN elements modified in the U3-region of the 3'-LTR of the vector plasmid suitable for being comprised in retroviral vectors. It is a specific advantage of both the modified 5'-promoter element and of the modified 3'-SIN element, which are preferably contained in retroviral vectors in combination with one another, to increase both the titer of viral particles as well as to increase the expression of a transgene in recipient cells, which transgene is arranged between the modified LTRs according to the invention.

### Annex to the application documents - subsequently filed sequences listing

SEQUENCE LISTING

<110>  Medinzinische Hochschule Hannover

<120>  Self-inactivating Gammaretroviral Vector

<130>  N1027-N-EP

<160>  5

<170>  PatentIn version 3.1

<210>  1
<211>  5124
<212>  DNA
<213>  artificial sequence

<220>
<223>  Gammaretroviral vector
<220>
<221>  promoter
<222>  (447)..(675)
<223>  RSV promoter, bases -233 to -1 relative to transcriptional start

<220>
<221>  R-U5
<222>  (676)..(820)
<223>  R and U5 region of the LTR

<220>
<221>  PBS
<222>  (821)..(837)
<223>  primer binding site

<220>

<221>  SD
<222>  (882)..(883)
<223>  splice donor

<220>
<221>  SFFV Promoter
<222>  (1314)..(1714)
<223>  internal promoter, spleen focus forming virus (SFFV) U3

<220>
<221>  gene
<222>  (1776)..(2495)
<223>  green fluorescent protein, cDNA

<220>
<221>  3' LTR
<222>  (2578)..(2872)
<223>  self-inactivating (SIN) LTR

<220>
<221>  SV40 USE
<222>  (2612)..(2655)
<223>  Simian virus 40 late mRNA upstream efficiency element

<220>
<221>  SV40 USE
<222>  (2662)..(2705)
<223>  Simian virus 40 late mRNA upstream efficiency element

<400>  1
ctgcctcgcg cgtttcggtg atgacggtga aaacctctga cacatgcagc tcccggagac      60
ggtcacagct tgtctgtaag cggatgccgg gagcagacaa gcccgtcagg gcgcgtcagc     120
gggtgttggc gggtgtcggg gcgcagccat gacccagtca cgtagcgata gttactatgc     180
ggcatcagag cagattgtac tgagagtgca ccatatgcgg tgtgaaatac cgcacagatg     240
cgtaaggaga aaataccgca tcaggcgcca ttcgccattc aggctgcgca actgttggga     300

agggcgatcg gtgcgggcct cttcgctatt acgccagctg gcgaaagggg gatgtgctgc 360
aaggcgatta agttgggtaa cgccagggtt ttcccagtca cgacgttgta aaacgacggc 420
cagtgaatta gtactctagc ttaagaatgt agtcttatgc aatactcttg tagtcttgca 480
acatggtaac gatgagttag caacatgcct tacaaggaga gaaaaagcac cgtgcatgcc 540
gattggtgga agtaaggtgg tacgatcgtg ccttattagg aaggcaacag acgggtctga 600
catggattgg acgaaccact gaattgccgc attgcagaga tattgtattt aagtgcctag 660
ctcgatacaa taaacgcgcc agtcctccga ttgactgcgt cgcccgggta cccgtattcc 720
caataaagcc tcttgctgtt tgcatccgaa tcgtggactc gctgatcctt gggagggtct 780
cctcagattg attgactgcc cacctcgggg gtctttcatt tggaggttcc accgagattt 840
ggagacccct gcccagggac caccgacccc cccgccggga ggtaagctgg ccagcggtcg 900
tttcgtgtct gtctctgtct ttgggcgtgt ttgtgccggc atctagtgtt tgcgcctgcg 960
tctgtactag ttggctaact agatctgtat ctggcggtcc cgcggaagaa ctgacgagtt 1020
cgtattcccg gccgcagccc ctgggagacg tcccagcggc ctcgggggcc cgttttgtgg 1080
cccattctgt atcagttaac ctacccgagt cggacttttt ggagctccgc cactgtccga 1140
ggggtacgtg gctttgttgg gggacgagag acagagacac ttcccgcccc cgtctgaatt 1200
tttgctttcg gttttacgcc gaaaccgcgc cgcgcgtctt gtctgctgca gcatcgttct 1260
gtgttgtctc tgtctgactg tgtttctgta tttgtctgaa aattagcggc ccgagctagc 1320
tgcagtaacg ccattttgca aggcatggaa aaataccaaa ccaagaatag agaagttcag 1380
atcaagggcg ggtacatgaa aatagctaac gttgggccaa acaggatatc tgcggtgagc 1440
agtttcggcc ccggcccggg gccaagaaca gatggtcacc gcagtttcgg ccccggcccg 1500
aggccaagaa cagatggtcc ccagatatgg cccaaccctc agcagtttct taagacccat 1560
cagatgtttc caggctcccc caaggacctg aaatgaccct gcgccttatt tgaattaacc 1620
aatcagcctg cttctcgctt ctgttcgcgc gcttctgctt cccgagctct ataaaagagc 1680
tcacaacccc tcactcggcg cgccagtcct ccgacagact gagtcggccg gtcgaatcaa 1740
gcttatgggc cgctctagcg ctaccggtcg ccaccatggt gagcaagggc gaggagctgt 1800
tcaccggggt ggtgcccatc ctggtcgagc tggacggcga cgtaaacggc cacaagttca 1860
gcgtgtccgg cgagggcgag ggcgatgcca cctacggcaa gctgaccctg aagttcatct 1920
gcaccaccgg caagctgccc gtgccctggc ccaccctcgt gaccaccctg acctacggcg 1980
tgcagtgctt cagccgctac cccgaccaca tgaagcagca cgacttcttc aagtccgcca 2040
tgcccgaagg ctacgtccag gagcgcacca tcttcttcaa ggacgacggc aactacaaga 2100
cccgcgccga ggtgaagttc gagggcgaca ccctggtgaa ccgcatcgag ctgaagggca 2160
tcgacttcaa ggaggacggc aacatcctgg ggcacaagct ggagtacaac tacaacagcc 2220
acaacgtcta tatcatggcc gacaagcaga agaacggcat caaggtgaac ttcaagatcc 2280
gccacaacat cgaggacggc agcgtgcagc tcgccgacca ctaccagcag aacaccccca 2340

tcggcgacgg ccccgtgctg ctgcccgaca accactacct gagcacccag tccgccctga 2400
gcaaagaccc caacgagaag cgcgatcaca tggtcctgct ggagttcgtg accgccgccg 2460
ggatcactct cggcatggac gagctgtaca agtaaagcgg ccgcgtcgac ggatccaagc 2520
ttaacacgag ccatagatag aataaaagat tttatttagt ctccagaaaa aggggggaat 2580
gaaagacccc accgctagcg atatcgaatt ctttatttgt gaaatttgtg atgctattgc 2640
tttatttgta accatgaatt ctttatttgt gaaatttgtg atgctattgt tttatttgta 2700
accatgaatt cacaacccct cactcggcgc gccagtcctc cgacagactg agtcgcccgg 2760
gtacccgtgt tctcaataaa ccctcttgca gttgcatccg actcgtggtc tcgctgttcc 2820
ttgggagggt ctcctctgag tgattgactg cccacctcgg gggtctttca ttctcgagag 2880
ctttggcgta atcatggtca tagctgtttc ctgtgtgaaa ttgttatccg ctcacaattc 2940
cacacaacat acgagccgga agcataaagt gtaaagcctg gggtgcctaa tgagtgagct 3000
aactcacatt aattgcgttg cgctcactgc ccgctttcca gtcgggaaac ctgtcgtgcc 3060
agctgcatta atgaatcggc caacgcgcgg ggagaggcgg tttgcgtatt gggcgctctt 3120
ccgcttcctc gctcactgac tcgctgcgct cggtcgttcg gctgcggcga gcggtatcag 3180
ctcactcaaa ggcggtaata cggttatcca cagaatcagg ggataacgca ggaaagaaca 3240
tgtgagcaaa aggccagcaa aaggccagga accgtaaaaa ggccgcgttg ctggcgtttt 3300
tccataggct ccgcccccct gacgagcatc acaaaaatcg acgctcaagt cagaggtggc 3360
gaaacccgac aggactataa agataccagg cgtttccccc tggaagctcc ctcgtgcgct 3420
ctcctgttcc gaccctgccg cttaccggat acctgtccgc ctttctccct tcgggaagcg 3480
tggcgctttc tcaatgctca cgctgtaggt atctcagttc ggtgtaggtc gttcgctcca 3540
agctgggctg tgtgcacgaa ccccccgttc agcccgaccg ctgcgcctta tccggtaact 3600
atcgtcttga gtccaacccg gtaagacacg acttatcgcc actggcagca gccactggta 3660
acaggattag cagagcgagg tatgtaggcg gtgctacaga gttcttgaag tggtggccta 3720
actacggcta cactagaagg acagtatttg gtatctgcgc tctgctgaag ccagttacct 3780
tcggaaaaag agttggtagc tcttgatccg gcaaacaaac caccgctggt agcggtggtt 3840
tttttgtttg caagcagcag attacgcgca gaaaaaaagg atctcaagaa gatcctttga 3900
tcttttctac ggggtctgac gctcagtgga acgaaaactc acgttaaggg attttggtca 3960
tgagattatc aaaaaggatc ttcacctaga tccttttaaa ttaaaaatga agttttaaat 4020
caatctaaag tatatatgag taaacttggt ctgacagtta ccaatgctta atcagtgagg 4080
cacctatctc agcgatctgt ctatttcgtt catccatagt tgcctgactc cccgtcgtgt 4140
agataactac gatacgggag ggcttaccat ctggccccag tgctgcaatg ataccgcgag 4200
acccacgctc accggctcca gatttatcag caataaacca gccagccgga agggccgagc 4260
gcagaagtgg tcctgcaact ttatccgcct ccatccagtc tattaattgt tgccgggaag 4320
ctagagtaag tagttcgcca gttaatagtt tgcgcaacgt tgttgccatt gctgctggca 4380
tcgtggtgtc acgctcgtcg tttggtatgg cttcattcag ctccggttcc caacgatcaa 4440

ggcgagttac atgatccccc atgttgtgca aaaaagcggt tagctccttc ggtcctccga 4500
tcgttgtcag aagtaagttg gccgcagtgt tatcactcat ggttatggca gcactgcata 4560
attctcttac tgtcatgcca tccgtaagat gcttttctgt gactggtgag tactcaacca 4620
agtcattctg agaatagtgt atgcggcgac cgagttgctc ttgcccggcg tcaatacggg 4680
ataataccgc gccacatagc agaactttaa aagtgctcat cattggaaaa cgttcttcgg 4740
ggcgaaaact ctcaaggatc ttaccgctgt tgagatccag ttcgatgtaa cccactcgtg 4800
cacccaactg atcttcagca tcttttactt tcaccagcgt ttctgggtga gcaaaaacag 4860
gaaggcaaaa tgccgcaaaa aagggaataa gggcgacacg gaaatgttga atactcatac 4920
tcttcctttt tcaatattat tgaagcattt atcagggtta ttgtctcatg agcggataca 4980
tatttgaatg tatttagaaa aataaacaaa taggggttcc gcgcacattt ccccgaaaag 5040
tgccacctga cgtctaagaa accattatta tcatgacatt aacctataaa aataggcgta 5100
tcacgaggcc ctttcgtctt caag 5124

<210> 2
<211> 5742
<212> DNA
<213> artificial sequence

<220>
<223> retroviral vector
<220>
<221> promoter
<222> (447)..(762)
<223> tetracyclin promoter "Tet"

<220>
<221> misc_feature
<222> (763)..(907)
<223>

<220>
<221> misc_feature
<222> (908)..(924)
<223> Primer binding site

<220>

<221>  misc_feature

<222>  (969)..(970)

<223>  SD = splice donor


<220>

<221>  promoter

<222>  (1401)..(1801)

<223>  SFFV promoter


<220>

<221>  CDS

<222>  (1863)..(2582)

<223>  GFP


<220>

<221>  misc_feature

<222>  (2621)..(3223)

<223>  PRE = post-transscriptional regulatory element (WHV), X protein d
       eleted


<220>

<221>  misc_feature

<222>  (3296)..(3485)

<223>  3´LTR, SIN


<400>  2
ctgcctcgcg cgtttcggtg atgacggtga aaacctctga cacatgcagc tcccggagac      60

ggtcacagct tgtctgtaag cggatgccgg gagcagacaa gcccgtcagg gcgcgtcagc     120

gggtgttggc gggtgtcggg gcgcagccat gacccagtca cgtagcgata gttactatgc     180

ggcatcagag cagattgtac tgagagtgca ccatatgcgg tgtgaaatac cgcacagatg     240

cgtaaggaga aaataccgca tcaggcgcca ttcgccattc aggctgcgca actgttggga     300

agggcgatcg gtgcgggcct cttcgctatt acgccagctg gcgaaagggg gatgtgctgc     360

aaggcgatta agttgggtaa cgccagggtt ttcccagtca cgacgttgta aaacgacggc     420

cagtgaatta gtactctagc ttaagcttct ttcacttttc tctgtcactg acagggagtg     480

gtaaactcga ctttcacttt tctctgtcac tgacagggag tggtaaactc gactttcact     540

tttctctgtc actgacaggg agtggtaaac tcgactttca cttttctctg tcactgacag     600

ggagtggtaa actcgacttt cacttttctc tgtcactgac agggagtggt aaactcgact     660

ttcacttttc tctgtcactg acagggagtg gtaaactcga gcgaatttag agcgcttctg     720

ctccccgagc tctataaaag agcccacaac ccctcactcg gggcgccagt cctccgattg     780

actgcgtcgc ccgggtaccc gtattcccaa taaagcctct tgctgtttgc atccgaatcg     840

tggactcgct gatccttggg agggtctcct cagattgatt gactgcccac ctcgggggtc     900

tttcatttgg aggttccacc gagatttgga gacccctgcc cagggaccac cgaccccccc     960

gccgggaggt aagctggcca gcggtcgttt cgtgtctgtc tctgtctttg ggcgtgtttg    1020

tgccggcatc tagtgtttgc gcctgcgtct gtactagttg gctaactaga tctgtatctg    1080

gcggtcccgc ggaagaactg acgagttcgt attcccggcc gcagcccctg ggagacgtcc    1140

cagcggcctc gggggcccgt tttgtggccc attctgtatc agttaaccta cccgagtcgg    1200

actttttgga gctccgccac tgtccgaggg gtacgtggct ttgttggggg acgagagaca    1260

gagacacttc ccgcccccgt ctgaattttt gctttcggtt ttacgccgaa accgcgccgc    1320

gcgtcttgtc tgctgcagca tcgttctgtg ttgtctctgt ctgactgtgt ttctgtattt    1380

gtctgaaaat tagcggcccg agctagctgc agtaacgcca ttttgcaagg catggaaaaa    1440

taccaaacca agaatagaga agttcagatc aagggcgggt acatgaaaat agctaacgtt    1500

gggccaaaca ggatatctgc ggtgagcagt ttcggccccg gcccggggcc aagaacagat    1560

ggtcaccgca gtttcggccc cggcccgagg ccaagaacag atggtcccca gatatggccc    1620

aaccctcagc agtttcttaa gacccatcag atgtttccag gctcccccaa ggacctgaaa    1680

tgaccctgcg ccttatttga attaaccaat cagcctgctt ctcgcttctg ttcgcgcgct    1740

tctgcttccc gagctctata aaagagctca caacccctca ctcggcgcgc cagtcctccg    1800

acagactgag tcggccggtc gaatcaagct tatgggccgc tctagcgcta ccggtcgcca    1860

cc atg gtg agc aag ggc gag gag ctg ttc acc ggg gtg gtg ccc atc       1907
   Met Val Ser Lys Gly Glu Glu Leu Phe Thr Gly Val Val Pro Ile
   1               5                   10                  15

ctg gtc gag ctg gac ggc gac gta aac ggc cac aag ttc agc gtg tcc      1955
Leu Val Glu Leu Asp Gly Asp Val Asn Gly His Lys Phe Ser Val Ser
                20                  25                  30

ggc gag ggc gag ggc gat gcc acc tac ggc aag ctg acc ctg aag ttc      2003
Gly Glu Gly Glu Gly Asp Ala Thr Tyr Gly Lys Leu Thr Leu Lys Phe
            35                  40                  45

atc tgc acc acc ggc aag ctg ccc gtg ccc tgg ccc acc ctc gtg acc      2051
Ile Cys Thr Thr Gly Lys Leu Pro Val Pro Trp Pro Thr Leu Val Thr
        50                  55                  60

acc ctg acc tac ggc gtg cag tgc ttc agc cgc tac ccc gac cac atg      2099
Thr Leu Thr Tyr Gly Val Gln Cys Phe Ser Arg Tyr Pro Asp His Met
    65                  70                  75

aag cag cac gac ttc ttc aag tcc gcc atg ccc gaa ggc tac gtc cag      2147
Lys Gln His Asp Phe Phe Lys Ser Ala Met Pro Glu Gly Tyr Val Gln
80                  85                  90                  95

gag cgc acc atc ttc ttc aag gac gac ggc aac tac aag acc cgc gcc     2195
Glu Arg Thr Ile Phe Phe Lys Asp Asp Gly Asn Tyr Lys Thr Arg Ala
                100                 105                 110

gag gtg aag ttc gag ggc gac acc ctg gtg aac cgc atc gag ctg aag     2243
Glu Val Lys Phe Glu Gly Asp Thr Leu Val Asn Arg Ile Glu Leu Lys
            115                 120                 125

ggc atc gac ttc aag gag gac ggc aac atc ctg ggg cac aag ctg gag     2291
Gly Ile Asp Phe Lys Glu Asp Gly Asn Ile Leu Gly His Lys Leu Glu
        130                 135                 140

tac aac tac aac agc cac aac gtc tat atc atg gcc gac aag cag aag     2339
Tyr Asn Tyr Asn Ser His Asn Val Tyr Ile Met Ala Asp Lys Gln Lys
    145                 150                 155

aac ggc atc aag gtg aac ttc aag atc cgc cac aac atc gag gac ggc     2387
Asn Gly Ile Lys Val Asn Phe Lys Ile Arg His Asn Ile Glu Asp Gly
160                 165                 170                 175

agc gtg cag ctc gcc gac cac tac cag cag aac acc ccc atc ggc gac     2435
Ser Val Gln Leu Ala Asp His Tyr Gln Gln Asn Thr Pro Ile Gly Asp
                180                 185                 190

ggc ccc gtg ctg ctg ccc gac aac cac tac ctg agc acc cag tcc gcc     2483
Gly Pro Val Leu Leu Pro Asp Asn His Tyr Leu Ser Thr Gln Ser Ala
            195                 200                 205

ctg agc aaa gac ccc aac gag aag cgc gat cac atg gtc ctg ctg gag     2531
Leu Ser Lys Asp Pro Asn Glu Lys Arg Asp His Met Val Leu Leu Glu
        210                 215                 220

ttc gtg acc gcc gcc ggg atc act ctc ggc atg gac gag ctg tac aag     2579
Phe Val Thr Ala Ala Gly Ile Thr Leu Gly Met Asp Glu Leu Tyr Lys
    225                 230                 235

taa agcggccgcg tcgacggatc ccccgggctg caggaattcg agcatcttac          2632

cgccatttat acccatattt gttctgtttt tcttgatttg ggtatacatt taaatgttaa   2692

taaaacaaaa tggtggggca atcatttaca tttttaggga tatgtaatta ctagttcagg   2752

tgtattgcca caagacaaac atgttaagaa actttcccgt tatttacgct ctgttcctgt   2812

taatcaacct ctggattaca aaatttgtga aagattgact gatattctta actatgttgc   2872

tccttttacg ctgtgtggat atgctgcttt aatgcctctg tatcatgcta ttgcttcccg   2932

tacggctttc gttttctcct ccttgtataa atcctggttg ctgtctcttt atgaggagtt   2992

gtggcccgtt gtccgtcaac gtggcgtggt gtgctctgtg tttgctgacg caacccccac   3052

tggctggggc attgccacca cctgtcaact cctttctggg actttcgctt tccccctccc   3112

gatcgccacg gcagaactca tcgccgcctg ccttgcccgc tgctggacag gggctaggtt   3172

gctgggcact gataattccg tggtgttgtc ggggaagctg acgtcctttc gaattcgata   3232

tcaagcttaa cacgagccat agatagaata aaagatttta tttagtctcc agaaaaaggg   3292

gggaatgaaa gaccccaccg ctagcgatat cgaattcaca acccctcact cggcgcgcca   3352

gtcctccgac agactgagtc gcccgggtac ccgtgttctc aataaaccct cttgcagttg   3412

catccgactc gtggtctcgc tgttccttgg gagggtctcc tctgagtgat tgactgccca   3472

cctcgggggt ctttcattct cgagagcttt ggcgtaatca tggtcatagc tgtttcctgt   3532

gtgaaattgt tatccgctca caattccaca caacatacga gccggaagca taaagtgtaa 3592
agcctggggt gcctaatgag tgagctaact cacattaatt gcgttgcgct cactgcccgc 3652
tttccagtcg ggaaacctgt cgtgccagct gcattaatga atcggccaac gcgcggggag 3712
aggcggtttg cgtattgggc gctcttccgc ttcctcgctc actgactcgc tgcgctcggt 3772
cgttcggctg cggcgagcgg tatcagctca ctcaaaggcg gtaatacggt tatccacaga 3832
atcaggggat aacgcaggaa agaacatgtg agcaaaaggc cagcaaaagg ccaggaaccg 3892
taaaaaggcc gcgttgctgg cgtttttcca taggctccgc ccccctgacg agcatcacaa 3952
aaatcgacgc tcaagtcaga ggtggcgaaa cccgacagga ctataaagat accaggcgtt 4012
tccccctgga agctccctcg tgcgctctcc tgttccgacc ctgccgctta ccggatacct 4072
gtccgccttt ctcccttcgg gaagcgtggc gctttctcaa tgctcacgct gtaggtatct 4132
cagttcggtg taggtcgttc gctccaagct gggctgtgtg cacgaacccc ccgttcagcc 4192
cgaccgctgc gccttatccg gtaactatcg tcttgagtcc aacccggtaa gacacgactt 4252
atcgccactg gcagcagcca ctggtaacag gattagcaga gcgaggtatg taggcggtgc 4312
tacagagttc ttgaagtggt ggcctaacta cggctacact agaaggacag tatttggtat 4372
ctgcgctctg ctgaagccag ttaccttcgg aaaaagagtt ggtagctctt gatccggcaa 4432
acaaaccacc gctggtagcg gtggtttttt tgtttgcaag cagcagatta cgcgcagaaa 4492
aaaaggatct caagaagatc ctttgatctt ttctacgggg tctgacgctc agtggaacga 4552
aaactcacgt taagggattt tggtcatgag attatcaaaa aggatcttca cctagatcct 4612
tttaaattaa aaatgaagtt ttaaatcaat ctaaagtata tatgagtaaa cttggtctga 4672
cagttaccaa tgcttaatca gtgaggcacc tatctcagcg atctgtctat ttcgttcatc 4732
catagttgcc tgactccccg tcgtgtagat aactacgata cgggagggct taccatctgg 4792
ccccagtgct gcaatgatac cgcgagaccc acgctcaccg gctccagatt tatcagcaat 4852
aaaccagcca gccggaaggg ccgagcgcag aagtggtcct gcaactttat ccgcctccat 4912
ccagtctatt aattgttgcc gggaagctag agtaagtagt tcgccagtta atagtttgcg 4972
caacgttgtt gccattgctg ctggcatcgt ggtgtcacgc tcgtcgtttg gtatggcttc 5032
attcagctcc ggttcccaac gatcaaggcg agttacatga tcccccatgt tgtgcaaaaa 5092
agcggttagc tccttcggtc ctccgatcgt tgtcagaagt aagttggccg cagtgttatc 5152
actcatggtt atggcagcac tgcataattc tcttactgtc atgccatccg taagatgctt 5212
ttctgtgact ggtgagtact caaccaagtc attctgagaa tagtgtatgc ggcgaccgag 5272
ttgctcttgc ccggcgtcaa tacgggataa taccgcgcca catagcagaa ctttaaaagt 5332
gctcatcatt ggaaaacgtt cttcggggcg aaaactctca aggatcttac cgctgttgag 5392
atccagttcg atgtaaccca ctcgtgcacc caactgatct tcagcatctt ttactttcac 5452
cagcgtttct gggtgagcaa aaacaggaag gcaaaatgcc gcaaaaaagg gaataagggc 5512
gacacggaaa tgttgaatac tcatactctt cctttttcaa tattattgaa gcatttatca 5572
gggttattgt ctcatgagcg gatacatatt tgaatgtatt tagaaaaata aacaaatagg 5632

ggttccgcgc acatttcccc gaaaagtgcc acctgacgtc taagaaacca ttattatcat   5692

gacattaacc tataaaaata ggcgtatcac gaggcccttt cgtcttcaag              5742

<210>  3

<211>  239

<212>  PRT

<213>  artificial sequence

<220>

<223>  retroviral vector

<220>

<221>  misc_feature

<222>  (763)..(907)

<223>

<220>

<221>  misc_feature

<222>  (908)..(924)

<223>  Primer binding site

<220>

<221>  misc_feature

<222>  (969)..(970)

<223>  SD = splice donor

<220>

<221>  misc_feature

<222>  (2621)..(3223)

<223>  PRE = post-transscriptional regulatory element (WHV), X protein d
       eleted

<220>

<221>  misc_feature

<222>  (3296)..(3485)

<223>  3´LTR, SIN

<400>  3

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  4

<211>  32

<212>  DNA

<213>  artificial sequence


<220>

<223>  primer

<400>  4

gctacttaag cttctttcac ttttctctgt ca                                    32

<210>  5

<211>  20

<212>  DNA

<213>  artificial sequence

<220>

<223>  primer

<400>  5
gagaacacgg gtacccgggc                                                  20

SEQUENCE LISTING

<110> Medinzinische Hochschule Hannover

<120> Self-inactivating Retroviral Vector

<130> N1027-N-EP

<140> EP06119359.5
<141> 2006-08-23

<160> 5

<170> PatentIn version 3.1

<210> 1
<211> 5124
<212> DNA
<213> artificial sequence

<220>
<223> Gammaretroviral vector
<220>
<221> promoter
<222> (447)..(675)
<223> RSV promoter, bases -233 to -1 relative to transcriptional start

<220>
<221> 5'UTR
<222> (676)..(820)
<223> R and U5 region of the LTR

<220>
<221> primer_bind
<222> (821)..(837)

<223>  primer binding site

<220>
<221>  misc_structure
<222>  (882)..(883)
<223>  splice donor

<220>
<221>  promoter
<222>  (1314)..(1714)
<223>  internal promoter, spleen focus forming virus (SFFV) U3

<220>
<221>  gene
<222>  (1776)..(2495)
<223>  green fluorescent protein, cDNA

<220>
<221>  3'UTR
<222>  (2578)..(2872)
<223>  3´LTR -  self-inactivating (SIN) LTR

<220>
<221>  misc_feature
<222>  (2612)..(2655)
<223>  SV40 USE - Simian virus 40 late mRNA upstream efficiency element

<220>
<221>  misc_feature
<222>  (2662)..(2705)
<223>  SV40 USE - Simian virus 40 late mRNA upstream efficiency element

<400>  1
ctgcctcgcg cgtttcggtg atgacggtga aaacctctga cacatgcagc tcccggagac      60
ggtcacagct tgtctgtaag cggatgccgg gagcagacaa gcccgtcagg gcgcgtcagc     120

gggtgttggc gggtgtcggg gcgcagccat gacccagtca cgtagcgata gttactatgc 180
ggcatcagag cagattgtac tgagagtgca ccatatgcgg tgtgaaatac cgcacagatg 240
cgtaaggaga aaataccgca tcaggcgcca ttcgccattc aggctgcgca actgttggga 300
agggcgatcg gtgcgggcct cttcgctatt acgccagctg gcgaaagggg gatgtgctgc 360
aaggcgatta agttgggtaa cgccagggtt ttcccagtca cgacgttgta aaacgacggc 420
cagtgaatta gtactctagc ttaagaatgt agtcttatgc aatactcttg tagtcttgca 480
acatggtaac gatgagttag caacatgcct tacaaggaga gaaaaagcac cgtgcatgcc 540
gattggtgga agtaaggtgg tacgatcgtg ccttattagg aaggcaacag acgggtctga 600
catggattgg acgaaccact gaattgccgc attgcagaga tattgtattt aagtgcctag 660
ctcgatacaa taaacgcgcc agtcctccga ttgactgcgt cgcccgggta cccgtattcc 720
caataaagcc tcttgctgtt tgcatccgaa tcgtggactc gctgatcctt gggagggtct 780
cctcagattg attgactgcc cacctcgggg gtctttcatt tggaggttcc accgagattt 840
ggagacccct gcccagggac caccgacccc cccgccggga ggtaagctgg ccagcggtcg 900
tttcgtgtct gtctctgtct ttgggcgtgt ttgtgccggc atctagtgtt tgcgcctgcg 960
tctgtactag ttggctaact agatctgtat ctggcggtcc cgcggaagaa ctgacgagtt 1020
cgtattcccg gccgcagccc ctgggagacg tcccagcggc ctcgggggcc cgttttgtgg 1080
cccattctgt atcagttaac ctacccgagt cggacttttt ggagctccgc cactgtccga 1140
ggggtacgtg gctttgttgg gggacgagag acagagacac ttcccgcccc cgtctgaatt 1200
tttgctttcg gttttacgcc gaaaccgcgc cgcgcgtctt gtctgctgca gcatcgttct 1260
gtgttgtctc tgtctgactg tgtttctgta tttgtctgaa aattagcggc ccgagctagc 1320
tgcagtaacg ccattttgca aggcatggaa aaataccaaa ccaagaatag agaagttcag 1380
atcaagggcg ggtacatgaa aatagctaac gttgggccaa acaggatatc tgcggtgagc 1440
agtttcggcc ccggcccggg gccaagaaca gatggtcacc gcagtttcgg ccccggcccg 1500
aggccaagaa cagatggtcc ccagatatgg cccaaccctc agcagtttct taagacccat 1560
cagatgtttc caggctcccc caaggacctg aaatgaccct gcgccttatt tgaattaacc 1620
aatcagcctg cttctcgctt ctgttcgcgc gcttctgctt cccgagctct ataaaagagc 1680
tcacaacccc tcactcggcg cgccagtcct ccgacagact gagtcggccg gtcgaatcaa 1740
gcttatgggc cgctctagcg ctaccggtcg ccaccatggt gagcaagggc gaggagctgt 1800
tcaccggggt ggtgcccatc ctggtcgagc tggacggcga cgtaaacggc cacaagttca 1860
gcgtgtccgg cgagggcgag ggcgatgcca cctacggcaa gctgaccctg aagttcatct 1920
gcaccaccgg caagctgccc gtgccctggc ccaccctcgt gaccaccctg acctacggcg 1980
tgcagtgctt cagccgctac cccgaccaca tgaagcagca cgacttcttc aagtccgcca 2040
tgcccgaagg ctacgtccag gagcgcacca tcttcttcaa ggacgacggc aactacaaga 2100
cccgcgccga ggtgaagttc gagggcgaca ccctggtgaa ccgcatcgag ctgaagggca 2160

tcgacttcaa ggaggacggc aacatcctgg ggcacaagct ggagtacaac tacaacagcc 2220
acaacgtcta tatcatggcc gacaagcaga agaacggcat caaggtgaac ttcaagatcc 2280
gccacaacat cgaggacggc agcgtgcagc tcgccgacca ctaccagcag aacaccccca 2340
tcggcgacgg ccccgtgctg ctgcccgaca accactacct gagcacccag tccgccctga 2400
gcaaagaccc caacgagaag cgcgatcaca tggtcctgct ggagttcgtg accgccgccg 2460
ggatcactct cggcatggac gagctgtaca agtaaagcgg ccgcgtcgac ggatccaagc 2520
ttaacacgag ccatagatag aataaaagat tttatttagt ctccagaaaa aggggggaat 2580
gaaagacccc accgctagcg atatcgaatt ctttatttgt gaaatttgtg atgctattgc 2640
tttatttgta accatgaatt ctttatttgt gaaatttgtg atgctattgt tttatttgta 2700
accatgaatt cacaacccct cactcggcgc gccagtcctc cgacagactg agtcgcccgg 2760
gtacccgtgt tctcaataaa ccctcttgca gttgcatccg actcgtggtc tcgctgttcc 2820
ttgggagggt ctcctctgag tgattgactg cccacctcgg gggtctttca ttctcgagag 2880
ctttggcgta atcatggtca tagctgtttc ctgtgtgaaa ttgttatccg ctcacaattc 2940
cacacaacat acgagccgga agcataaagt gtaaagcctg gggtgcctaa tgagtgagct 3000
aactcacatt aattgcgttg cgctcactgc ccgctttcca gtcgggaaac ctgtcgtgcc 3060
agctgcatta atgaatcggc caacgcgcgg ggagaggcgg tttgcgtatt gggcgctctt 3120
ccgcttcctc gctcactgac tcgctgcgct cggtcgttcg gctgcggcga gcggtatcag 3180
ctcactcaaa ggcggtaata cggttatcca cagaatcagg ggataacgca ggaaagaaca 3240
tgtgagcaaa aggccagcaa aaggccagga accgtaaaaa ggccgcgttg ctggcgtttt 3300
tccataggct ccgcccccct gacgagcatc acaaaaatcg acgctcaagt cagaggtggc 3360
gaaacccgac aggactataa agataccagg cgtttccccc tggaagctcc ctcgtgcgct 3420
ctcctgttcc gaccctgccg cttaccggat acctgtccgc ctttctccct tcgggaagcg 3480
tggcgctttc tcaatgctca cgctgtaggt atctcagttc ggtgtaggtc gttcgctcca 3540
agctgggctg tgtgcacgaa ccccccgttc agcccgaccg ctgcgcctta tccggtaact 3600
atcgtcttga gtccaacccg gtaagacacg acttatcgcc actggcagca gccactggta 3660
acaggattag cagagcgagg tatgtaggcg gtgctacaga gttcttgaag tggtggccta 3720
actacggcta cactagaagg acagtatttg gtatctgcgc tctgctgaag ccagttacct 3780
tcggaaaaag agttggtagc tcttgatccg gcaaacaaac caccgctggt agcggtggtt 3840
tttttgtttg caagcagcag attacgcgca gaaaaaaagg atctcaagaa gatcctttga 3900
tcttttctac ggggtctgac gctcagtgga acgaaaactc acgttaaggg attttggtca 3960
tgagattatc aaaaaggatc ttcacctaga tccttttaaa ttaaaaatga agttttaaat 4020
caatctaaag tatatatgag taaacttggt ctgacagtta ccaatgctta atcagtgagg 4080
cacctatctc agcgatctgt ctatttcgtt catccatagt tgcctgactc cccgtcgtgt 4140
agataactac gatacgggag ggcttaccat ctggccccag tgctgcaatg ataccgcgag 4200
acccacgctc accggctcca gatttatcag caataaacca gccagccgga agggccgagc 4260

gcagaagtgg tcctgcaact ttatccgcct ccatccagtc tattaattgt tgccgggaag 4320
ctagagtaag tagttcgcca gttaatagtt tgcgcaacgt tgttgccatt gctgctggca 4380
tcgtggtgtc acgctcgtcg tttggtatgg cttcattcag ctccggttcc caacgatcaa 4440
ggcgagttac atgatccccc atgttgtgca aaaaagcggt tagctccttc ggtcctccga 4500
tcgttgtcag aagtaagttg gccgcagtgt tatcactcat ggttatggca gcactgcata 4560
attctcttac tgtcatgcca tccgtaagat gcttttctgt gactggtgag tactcaacca 4620
agtcattctg agaatagtgt atgcggcgac cgagttgctc ttgcccggcg tcaatacggg 4680
ataataccgc gccacatagc agaactttaa aagtgctcat cattggaaaa cgttcttcgg 4740
ggcgaaaact ctcaaggatc ttaccgctgt tgagatccag ttcgatgtaa cccactcgtg 4800
cacccaactg atcttcagca tcttttactt tcaccagcgt ttctgggtga gcaaaaacag 4860
gaaggcaaaa tgccgcaaaa aagggaataa gggcgacacg gaaatgttga atactcatac 4920
tcttcctttt tcaatattat tgaagcattt atcagggtta ttgtctcatg agcggataca 4980
tatttgaatg tatttagaaa aataaacaaa taggggttcc gcgcacattt ccccgaaaag 5040
tgccacctga cgtctaagaa accattatta tcatgacatt aacctataaa aataggcgta 5100
tcacgaggcc ctttcgtctt caag 5124

<210> 2
<211> 5742
<212> DNA
<213> artificial sequence

<220>
<223> retroviral vector
<220>
<221> promoter
<222> (447)..(762)
<223> tetracyclin promoter "Tet"

<220>
<221> misc_feature
<222> (763)..(907)
<223> R and U5 regions

<220>
<221> misc_feature

<222>  (908)..(924)
<223>  Primer binding site


<220>
<221>  misc_feature
<222>  (969)..(970)
<223>  SD = splice donor


<220>
<221>  promoter
<222>  (1401)..(1801)
<223>  SFFV promoter


<220>
<221>  CDS
<222>  (1863)..(2582)
<223>  GFP


<220>
<221>  misc_feature
<222>  (2621)..(3223)
<223>  PRE = post-transscriptional regulatory element (WHV), X protein d
       eleted


<220>
<221>  misc_feature
<222>  (3296)..(3485)
<223>  3´LTR, SIN


<400>  2
ctgcctcgcg cgtttcggtg atgacggtga aaacctctga cacatgcagc tcccggagac      60

ggtcacagct tgtctgtaag cggatgccgg gagcagacaa gcccgtcagg gcgcgtcagc     120

gggtgttggc gggtgtcggg gcgcagccat gacccagtca cgtagcgata gttactatgc     180

ggcatcagag cagattgtac tgagagtgca ccatatgcgg tgtgaaatac cgcacagatg     240

cgtaaggaga aaataccgca tcaggcgcca ttcgccattc aggctgcgca actgttggga     300

agggcgatcg gtgcgggcct cttcgctatt acgccagctg gcgaaagggg gatgtgctgc 360

aaggcgatta agttgggtaa cgccagggtt ttcccagtca cgacgttgta aaacgacggc 420

cagtgaatta gtactctagc ttaagcttct ttcacttttc tctgtcactg acagggagtg 480

gtaaactcga ctttcacttt tctctgtcac tgacagggag tggtaaactc gactttcact 540

tttctctgtc actgacaggg agtggtaaac tcgactttca cttttctctg tcactgacag 600

ggagtggtaa actcgacttt cacttttctc tgtcactgac agggagtggt aaactcgact 660

ttcacttttc tctgtcactg acagggagtg gtaaactcga gcgaatttag agcgcttctg 720

ctccccgagc tctataaaag agcccacaac ccctcactcg gggcgccagt cctccgattg 780

actgcgtcgc ccgggtaccc gtattcccaa taaagcctct tgctgtttgc atccgaatcg 840

tggactcgct gatccttggg agggtctcct cagattgatt gactgcccac ctcgggggtc 900

tttcatttgg aggttccacc gagatttgga gacccctgcc cagggaccac cgaccccccc 960

gccgggaggt aagctggcca gcggtcgttt cgtgtctgtc tctgtctttg ggcgtgtttg 1020

tgccggcatc tagtgtttgc gcctgcgtct gtactagttg gctaactaga tctgtatctg 1080

gcggtcccgc ggaagaactg acgagttcgt attcccggcc gcagcccctg ggagacgtcc 1140

cagcggcctc gggggcccgt tttgtggccc attctgtatc agttaaccta cccgagtcgg 1200

actttttgga gctccgccac tgtccgaggg gtacgtggct ttgttggggg acgagagaca 1260

gagacacttc ccgcccccgt ctgaattttt gctttcggtt ttacgccgaa accgcgccgc 1320

gcgtcttgtc tgctgcagca tcgttctgtg ttgtctctgt ctgactgtgt ttctgtattt 1380

gtctgaaaat tagcggcccg agctagctgc agtaacgcca ttttgcaagg catggaaaaa 1440

taccaaacca agaatagaga agttcagatc aagggcgggt acatgaaaat agctaacgtt 1500

gggccaaaca ggatatctgc ggtgagcagt ttcggccccg gcccggggcc aagaacagat 1560

ggtcaccgca gtttcggccc cggcccgagg ccaagaacag atggtcccca gatatggccc 1620

aaccctcagc agtttcttaa gacccatcag atgtttccag gctcccccaa ggacctgaaa 1680

tgaccctgcg ccttatttga attaaccaat cagcctgctt ctcgcttctg ttcgcgcgct 1740

tctgcttccc gagctctata aaagagctca caacccctca ctcggcgcgc cagtcctccg 1800

acagactgag tcggccggtc gaatcaagct tatgggccgc tctagcgcta ccggtcgcca 1860

cc atg gtg agc aag ggc gag gag ctg ttc acc ggg gtg gtg ccc atc 1907
Met Val Ser Lys Gly Glu Glu Leu Phe Thr Gly Val Val Pro Ile
1               5                   10                  15

ctg gtc gag ctg gac ggc gac gta aac ggc cac aag ttc agc gtg tcc 1955
Leu Val Glu Leu Asp Gly Asp Val Asn Gly His Lys Phe Ser Val Ser
                20                  25                  30

ggc gag ggc gag ggc gat gcc acc tac ggc aag ctg acc ctg aag ttc 2003
Gly Glu Gly Glu Gly Asp Ala Thr Tyr Gly Lys Leu Thr Leu Lys Phe
            35                  40                  45

atc tgc acc acc ggc aag ctg ccc gtg ccc tgg ccc acc ctc gtg acc 2051
Ile Cys Thr Thr Gly Lys Leu Pro Val Pro Trp Pro Thr Leu Val Thr
        50                  55                  60

acc ctg acc tac ggc gtg cag tgc ttc agc cgc tac ccc gac cac atg 2099

Thr Leu Thr Tyr Gly Val Gln Cys Phe Ser Arg Tyr Pro Asp His Met
    65                  70                  75

aag cag cac gac ttc ttc aag tcc gcc atg ccc gaa ggc tac gtc cag      2147
Lys Gln His Asp Phe Phe Lys Ser Ala Met Pro Glu Gly Tyr Val Gln
80                  85                  90                  95

gag cgc acc atc ttc ttc aag gac gac ggc aac tac aag acc cgc gcc      2195
Glu Arg Thr Ile Phe Phe Lys Asp Asp Gly Asn Tyr Lys Thr Arg Ala
                100                 105                 110

gag gtg aag ttc gag ggc gac acc ctg gtg aac cgc atc gag ctg aag      2243
Glu Val Lys Phe Glu Gly Asp Thr Leu Val Asn Arg Ile Glu Leu Lys
            115                 120                 125

ggc atc gac ttc aag gag gac ggc aac atc ctg ggg cac aag ctg gag      2291
Gly Ile Asp Phe Lys Glu Asp Gly Asn Ile Leu Gly His Lys Leu Glu
        130                 135                 140

tac aac tac aac agc cac aac gtc tat atc atg gcc gac aag cag aag      2339
Tyr Asn Tyr Asn Ser His Asn Val Tyr Ile Met Ala Asp Lys Gln Lys
    145                 150                 155

aac ggc atc aag gtg aac ttc aag atc cgc cac aac atc gag gac ggc      2387
Asn Gly Ile Lys Val Asn Phe Lys Ile Arg His Asn Ile Glu Asp Gly
160                 165                 170                 175

agc gtg cag ctc gcc gac cac tac cag cag aac acc ccc atc ggc gac      2435
Ser Val Gln Leu Ala Asp His Tyr Gln Gln Asn Thr Pro Ile Gly Asp
                180                 185                 190

ggc ccc gtg ctg ctg ccc gac aac cac tac ctg agc acc cag tcc gcc      2483
Gly Pro Val Leu Leu Pro Asp Asn His Tyr Leu Ser Thr Gln Ser Ala
            195                 200                 205

ctg agc aaa gac ccc aac gag aag cgc gat cac atg gtc ctg ctg gag      2531
Leu Ser Lys Asp Pro Asn Glu Lys Arg Asp His Met Val Leu Leu Glu
        210                 215                 220

ttc gtg acc gcc gcc ggg atc act ctc ggc atg gac gag ctg tac aag      2579
Phe Val Thr Ala Ala Gly Ile Thr Leu Gly Met Asp Glu Leu Tyr Lys
    225                 230                 235

taa agcggccgcg tcgacggatc ccccgggctg caggaattcg agcatcttac      2632

cgccatttat acccatattt gttctgtttt tcttgatttg ggtatacatt taaatgttaa      2692

taaaacaaaa tggtggggca atcatttaca tttttaggga tatgtaatta ctagttcagg      2752

tgtattgcca caagacaaac atgttaagaa actttcccgt tatttacgct ctgttcctgt      2812

taatcaacct ctggattaca aaatttgtga aagattgact gatattctta actatgttgc      2872

tccttttacg ctgtgtggat atgctgcttt aatgcctctg tatcatgcta ttgcttcccg      2932

tacggctttc gttttctcct ccttgtataa atcctggttg ctgtctcttt atgaggagtt      2992

gtggcccgtt gtccgtcaac gtggcgtggt gtgctctgtg tttgctgacg caacccccac      3052

tggctggggc attgccacca cctgtcaact cctttctggg actttcgctt tccccctccc      3112

gatcgccacg gcagaactca tcgccgcctg ccttgcccgc tgctggacag gggctaggtt      3172

gctgggcact gataattccg tggtgttgtc ggggaagctg acgtcctttc gaattcgata      3232

tcaagcttaa cacgagccat agatagaata aaagatttta tttagtctcc agaaaaaggg      3292

gggaatgaaa gaccccaccg ctagcgatat cgaattcaca acccctcact cggcgcgcca      3352

gtcctccgac agactgagtc gcccgggtac ccgtgttctc aataaaccct cttgcagttg 3412
catccgactc gtggtctcgc tgttccttgg gagggtctcc tctgagtgat tgactgccca 3472
cctcgggggt ctttcattct cgagagcttt ggcgtaatca tggtcatagc tgtttcctgt 3532
gtgaaattgt tatccgctca caattccaca caacatacga gccggaagca taaagtgtaa 3592
agcctggggt gcctaatgag tgagctaact cacattaatt gcgttgcgct cactgcccgc 3652
tttccagtcg ggaaacctgt cgtgccagct gcattaatga atcggccaac gcgcggggag 3712
aggcggtttg cgtattgggc gctcttccgc ttcctcgctc actgactcgc tgcgctcggt 3772
cgttcggctg cggcgagcgg tatcagctca ctcaaaggcg gtaatacggt tatccacaga 3832
atcaggggat aacgcaggaa agaacatgtg agcaaaaggc cagcaaaagg ccaggaaccg 3892
taaaaaggcc gcgttgctgg cgtttttcca taggctccgc ccccctgacg agcatcacaa 3952
aaatcgacgc tcaagtcaga ggtggcgaaa cccgacagga ctataaagat accaggcgtt 4012
tccccctgga agctccctcg tgcgctctcc tgttccgacc ctgccgctta ccggatacct 4072
gtccgccttt ctcccttcgg gaagcgtggc gctttctcaa tgctcacgct gtaggtatct 4132
cagttcggtg taggtcgttc gctccaagct gggctgtgtg cacgaacccc ccgttcagcc 4192
cgaccgctgc gccttatccg gtaactatcg tcttgagtcc aacccggtaa gacacgactt 4252
atcgccactg gcagcagcca ctggtaacag gattagcaga gcgaggtatg taggcggtgc 4312
tacagagttc ttgaagtggt ggcctaacta cggctacact agaaggacag tatttggtat 4372
ctgcgctctg ctgaagccag ttaccttcgg aaaaagagtt ggtagctctt gatccggcaa 4432
acaaaccacc gctggtagcg gtggtttttt tgtttgcaag cagcagatta cgcgcagaaa 4492
aaaaggatct caagaagatc ctttgatctt ttctacgggg tctgacgctc agtggaacga 4552
aaactcacgt taagggattt tggtcatgag attatcaaaa aggatcttca cctagatcct 4612
tttaaattaa aaatgaagtt ttaaatcaat ctaaagtata tatgagtaaa cttggtctga 4672
cagttaccaa tgcttaatca gtgaggcacc tatctcagcg atctgtctat ttcgttcatc 4732
catagttgcc tgactccccg tcgtgtagat aactacgata cgggagggct taccatctgg 4792
ccccagtgct gcaatgatac cgcgagaccc acgctcaccg gctccagatt tatcagcaat 4852
aaaccagcca gccggaaggg ccgagcgcag aagtggtcct gcaactttat ccgcctccat 4912
ccagtctatt aattgttgcc gggaagctag agtaagtagt tcgccagtta atagtttgcg 4972
caacgttgtt gccattgctg ctggcatcgt ggtgtcacgc tcgtcgtttg gtatggcttc 5032
attcagctcc ggttcccaac gatcaaggcg agttacatga tcccccatgt tgtgcaaaaa 5092
agcggttagc tccttcggtc ctccgatcgt tgtcagaagt aagttggccg cagtgttatc 5152
actcatggtt atggcagcac tgcataattc tcttactgtc atgccatccg taagatgctt 5212
ttctgtgact ggtgagtact caaccaagtc attctgagaa tagtgtatgc ggcgaccgag 5272
ttgctcttgc ccggcgtcaa tacgggataa taccgcgcca catagcagaa ctttaaaagt 5332
gctcatcatt ggaaaacgtt cttcggggcg aaaactctca aggatcttac cgctgttgag 5392
atccagttcg atgtaaccca ctcgtgcacc caactgatct tcagcatctt ttactttcac 5452

cagcgtttct gggtgagcaa aaacaggaag gcaaaatgcc gcaaaaaagg gaataagggc 5512
gacacggaaa tgttgaatac tcatactctt cctttttcaa tattattgaa gcatttatca 5572
gggttattgt ctcatgagcg gatacatatt tgaatgtatt tagaaaaata aacaaatagg 5632
ggttccgcgc acatttcccc gaaaagtgcc acctgacgtc taagaaacca ttattatcat 5692
gacattaacc tataaaaata ggcgtatcac gaggcccttt cgtcttcaag 5742

<210> 3
<211> 239
<212> PRT
<213> artificial sequence

<220>
<223> retroviral vector

<400> 3

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210> 4

<211> 32

<212> DNA

<213> artificial sequence

<220>

<223> primer

<400> 4
gctacttaag cttctttcac ttttctctgt ca 32

<210> 5

<211> 20

<212> DNA

<213> artificial sequence

<220>

<223> primer

<400> 5
gagaacacgg gtacccgggc 20

## Description

### Self-inactivating Retroviral Vector

The present invention relates to retroviral vectors, especially to self-inactivating (SIN) lentiviral and gamma (γ)-retroviral vectors, suitable for producing viral particles at high titers, which can be used for efficient gene transfer into mammalian cells, organs or organisms, e.g. for gene therapy.

More specifically, the present invention provides modified 5'-promoter elements and modified 3'-SIN elements suitable for being comprised in retroviral vectors. It is a specific advantage of both the modified 5'-promoter element and of the modified 3'-SIN element, which may be contained in retroviral vectors separately, but preferably in combination with one another, to increase the titer of viral particles obtainable in packaging cells. The 3'-element also increases the expression of a transgene in recipient cells, which transgene is arranged between the SIN-elements according to the invention.

Furthermore, the 3'-element prevents transcriptional read-through from an integrated lentiviral or gammaretroviral vector into a downstream located cellular gene.

### State of the art

Retroviruses have been widely used as gene delivery tools. Because the retroviral genome inserts into the host cell genome following infection, it can be utilized as a permanent gene delivery vehicle. This key characteristic has been maintained in all the different types of replication incompetent retroviral vectors that have been designed. It allows the retroviral genome to be maintained for the life of the cell (Baum et al., Blood 101, 2099-2114 (2003); Thomas et al., Nature Rev Genet 4, 346-358 (2003).

Upon infection, the retrovirus introduces its RNA into the cytoplasm of a cell along with the reverse transcriptase enzyme. The RNA template is then reverse transcribed into a linear, double stranded cDNA that contains the virus-derived genetic instructions. Integration of the viral DNA into the host cell genome makes the infection permanent. The retrovirus replication strategy is designed for long-term persisting infection since the virus is spread both vertically (from parent cell to daughter cells via the provirus) as well as horizontally (from cell to cell via virions). Simple gammaretroviruses such as murine leukemia viruses (MLVs) do not kill the infected cell. The lentiviruses (*e.g.*, HIV-1) are complex retroviruses that have regulatory and structural elements in addition to the standard (gag-pol-env) elements, many of which have cytotoxic features. A retroviral (such as gammaretroviral or lentiviral) vector is designed to avoid the transfer and expression of toxic or immunogenic viral gene remnants in the target cell population (Baum et al., 2003; Hildinger et al., Journal of Virology 73, 4083-4089 (1999); Thomas et al., 2003).

To produce retroviral vectors in so-called packaging cells, at least 3 different plasmids are co-transfected, one encoding the retroviral gag-pol polyprotein, one encoding the env protein, and one encoding the vector mRNA that is to be packaged in newly formed particles. The uptake of the retroviral mRNA into the particles occurs in the cytoplasm of infected cells and is mediated by a specific packaging signal (Ψ) of the RNA that interacts with the nucleocapsid domain of the retroviral precursor protein called Gag, resulting in self-assembly of particles. In the case of simple gammaretroviral vectors derived from MLVs, Ψ does not overlap with viral coding regions (Hildinger et al., 1999). Upon budding through the cytoplasmic membrane, virus-encoded Env glycoproteins are incorporated into the membrane surrounding the viral particle.

To express the Ψ+ mRNA in packaging cells, the plasmids encoding the vector RNA need to be equipped with a 5'-promoter that initiates transcription at the first base of the R region and generates high levels of mRNA. 5'-promoters that are typically used for the generation of gammaretroviral mRNA in packaging cells are either an U3 region derived from MLV or the immediate early promoter of the human cytomegalovirus (CMV). The 5' promoter sequence itself will not be part of the retroviral mRNA and is therefore not present in the proviral DNA genome found in retrovirally transduced target cells.

State-of-the-art gammaretroviral vectors do not contain remnants of viral gag, pol or env genes (Hildinger et al., 1999). The posttranscriptional regulatory element (PRE) of woodchuck hepatitis virus (WHV) is useful in these vectors for enhancement of titers and expression (Schambach et al., Molecular Therapy 2, 435-445 (2000)).

Gammaretroviral vectors containing wild-type enhancer-promoter sequences in the U3 region of the LTRs may upregulate neighboring cellular alleles by insertional mutagenesis. This may lead to cancer or leukemia and represents a dose-limiting side effect for the clinical use of retroviral gene transfer technology (Baum et al., 2003; Hacein-Bey-Abina et al., Science 302, 415-419 (2003); Li et al., Science 296, 497 (2002); Modlich et al., Blood 105, 4235-4246 (2005)).

If the enhancer-promoter sequences from the U3 region of the LTRs are deleted, so-called self-inactivating (SIN) retroviral vectors can be generated in which the transcriptional control of the transgene product depends on an internal promoter that is located between the LTRs (Yu et al., Proceedings of the National Academy of Sciences of the United States of America 83, 3194-3198 (1986)). These vectors have a reduced risk of insertional mutagenesis. However, their infectious titers are significantly lower (more than 10-fold) than those achieved with conventional vectors (Kraunus et al., Gene Therapy 11, 1568-1578 (2004); Werner et al., Gene Therapy 9, 992-1000 (2004); Yu et al., 1986). This represents a major limitation for their clinical use. Many primary cells that are of great interest for human gene therapy can only be sufficiently transduced if the vector preparation has a high titer (more than 10⁶ transducing units per ml of producer cell supernatant). Increasing the titer of a vector preparation also reduces the costs of vector production. The increase in titer should be achieved without increasing the amount of plasmid transfected into packaging cells, because plasmid contaminations of vector preparations represent a potential safety-limitation.

The deletion of U3-sequences as present in state-of-the-art SIN vectors impedes retroviral transcriptional termination and polyadenylation (Furger et al., Journal of Virology 75, 11735-11746 (2001)). This may contribute to low titers and also reduce transgene expression in target cells. Insufficient termination of transcription also increases the risk of insertional readthrough and splicing of mRNA into downstream located cellular genes (Kustikova et al., Science 308, 1171-1174 (2005); Li et al., 2002).

Dull et al. (Journal of Virology 72 (11), 8463 - 8471 (1998)) analyze the minimal components required for a lentiviral vector used for nucleic acid transfer. For production of viral particles, genes essential for replication and packaging are separate on at least two separate vectors. Dull et al. show that the tat gene, which is crucial for HIV replication could be deleted from the lentiviral vector, resulting in vector particles made in the absence of tat, having a 10 - 20- fold reduced transducing activity. Activity levels could be improved by replacing the HIV sequence in the 5'-LTR of the transfer construct by strong constitutive promoters, when producing vectors without tat gene product.

Inducible promoters have also been shown to mediate high titers of lentiviral vectors (Xu et al., Mol. Ther. 97-104 (2001)), but their utility for gammaretroviral vectors remained unknown.

Zaiss et al. (Journal of Virology, 7209 - 7219 (2002)) analyze the importance of the 3' poly-A signal in lentiviral vectors used for delivery of nucleic acid sequences into mammalian cells in respect of the activation of sequences downstream the integration site. Further it was found that SIN vectors of MLV and HIV-1 frequently cause 3' RNA readthrough, emphasizing the importance of poly-A signals of viral origin for gene activation. Further, it was found that modification of the poly-A signal influenced the titer of the vector, suggesting that the addition of a strong poly-A signal increases vector efficiency when using MLV. Further, for transient transfections, reporter gene transcription was found to be increased by a stronger poly-A signal. However, Zaiss et al. did not report a functional retroviral vector with improved polyadenylation because they inserted the poly-A signal in 3' of the 3'-LTR.

Further, the effect of the HIV-upstream enhancer (HIV-USE)-element (wild-type except for some missing nucleotides in the 5' region of the USE-element) is cited by Zaiss et al. as leading to an increase in vector titer for HIV-SIN vectors.

Valsamakis et al. (Molecular and Cellular Biology, 3699-3705 (1992) show that the distance between the 5'-cap element and the HIV-poly-adenylation signal could be reduced to at least 140 nucleotides in place of the previously disclosed requirement for a spacing by at least 250 nucleotides. However, the sequences arranged between nucleotides 56 and 93 upstream the poly-adenylation signal were required for efficient *in vivo* poly-adenylation as well as for efficient cleavage and poly-adenylation *in vitro.* The region lying between 56 and 93 bases upstream the poly-adenylation signal is characterized as a USE-element which may be participating in the control of poly-adenylation efficiency, e.g. tissue specific poly-adenylation.

### Object of the invention

Deficiencies of known retroviral vectors include the higher risk of formation of replication competent retrovirus (RCR) when using wild-type LTR sequences, the proneness of wild-type LTR to induce insertional mutagenesis, possibly up-regulating neighbouring sequences, which may even cause leukemia in recipients as shown by Baum et al. (Blood, 101, No. 6, pages 2099-2114 (2003)) and low infectious titers when using SIN sequences instead of wild-type LTRs. Therefore, it is an object of the present invention to provide sequence elements suitable for retroviral vectors that allow for the production of viral particles containing such vectors at high infectious titers and, concurrently, allowing a high activity of the transgene contained in the vector. Further, it is an object of the present invention to provide improved vectors containing such sequence elements, which allow the production of viral particles at high titers as well as the efficient transfection of target cells and good activity of the transgene while strongly reducing the risk of insufficient transcriptional termination. Further, it is an object of the present invention to provide a method for transducing mammalian cells, organs and organisms using a vector comprising said sequence elements, as well as providing the resultant mammalian cells, organs and organisms.

### General description of the invention

In general, the invention relates to retroviral vectors, especially to lentiviral and gammaretroviral vectors, suitable for producing viral particles at high titers, which can be used for efficient gene transfer into mammalian cells, organs or organisms, e.g. for gene therapy. More specifically, the present invention provides modified 5'-promoter elements in the U3-region of the 5'-LTR of the gammaretroviral vector plasmid and 3'-SIN elements modified in the U3-region of the 3'-LTR of the gammaretroviral vector plasmid or lentiviral vector plasmid suitable for being comprised in retroviral vectors. It is a specific advantage of both the modified 5'-promoter element and of the modified 3'-SIN element, which can be contained independently in gammaretroviral vectors or lentiviral vectors and which are preferably contained in combination with one another, to increase both the titer of viral particles in packaging cells as well as to increase the expression of a transgene in recipient cells, which transgene is arranged between the modified LTRs according to the invention.

Accordingly, the present invention achieves the above-mentioned objects by providing a retroviral vector comprising a 5'-promoter element which dominates a potential internal promoter of a retroviral vector in transfected packaging cells. This promoter is to replace the U3-element of the 5'-LTR and drives transcription of full-length mRNA suitable for packaging into viral particles, e.g. containing the retroviral packaging signal ψ.

Generally, the present invention relates to improved retroviral vectors, preferably gamma-retroviral vectors derived from MLV and lentiviral vectors derived from HIV. Generic vectors according to the state of art use SIN-elements instead of wild-type LTRs and, preferably, do not contain sequences derived from the viral gag, pol, or env genes. However, the post-transcriptional regulatory element of Woodchuck Hepatitis Virus (WPRE) is a potential component of the vectors according to the invention, as WPRE enhances the titer of infectious viral particles and expression levels of the transgene in transduced target cells. However, presence of the WPRE is not essential in vectors according to the invention to obtain high viral titers in packaging cells or for expression in target cells.

To-date, SIN-elements have an improved biosafety by the deletion of the strong enhancer-promoter element in the U3-region of the LTR. During reverse transcription within the target cell, the deletion of the U3-region of the 3'-LTR is transferred to the corresponding section of the 5'-LTR, which deletion is sufficient to abolish the transcriptional activity of the LTR promoter. As a result, transcription of full-length vector RNA is eliminated in transduced cells, concurrently reducing the possibility of unintentional activation of adjacent cellular gene sequences by enhancer-promoter activity of the LTR or readthrough. The deletion of an approx. 400 base pair segment of the 3' U3-region, resulting in its transcriptional inactivation of the proviral LTR in infected cells, increases biosafety, but reduces infectious titers at least ten-fold.

Preferably, the inventive vector comprising a 5'-LTR, containing the novel promoter element in its U3-region, is combined with an upstream enhancer element (USE-element) to be inserted into the U3-region of the 3'-LTR, preferably also of SIN-architecture.

The novel promoter element serves to efficiently express full-length mRNA containing the transgene, while the preferred embodiment, comprising the USE-element in the U3-region of the 3'-LTR, leads to further enhancement of viral titers, combined with higher transgene expression in transduced cells and, in accordance with the SIN-nature of the LTRs, to a further reduction of the risk of insertional mutagenesis by prevention of transcriptional read-through.

In general, a novel 5'-promoter element for use in the proviral SIN vector plasmid is derivable from Rous Sarcoma Virus (RSV) promoter, preferably containing additional enhancer sequences of Simian Virus 40 (SV40), inserted upstream of the RSV promoter. When transfecting retroviral packaging cells, synthesis of genomic full-length RNA of the SIN-vector is then driven by the novel promoter element, namely the RSV promoter (P_{RSV}), additionally comprising enhancer sequences derived from SV40. Because of the location of the novel promoter element in upstream from the first nucleotide of the retroviral RNA expressed in packaging cells, the novel promoter element will not be part of the sequence comprising the transgene to be integrated into the target cells.

Alternatively, the Rous Sarcoma Virus promoter can be replaced by a tetracycline-inducible promoter, inserted upstream of the promoter (Pₜₑₜ). The embodiments represented by P_{RSV} and Pₜₑₜ are referred to as the promoter according to the invention.

The USE-element to be contained in the 3'-LTR of the inventive vector, preferably in combination with the novel promoter element to be contained in the 5'-LTR, preferably consists of an artificial direct repeating element of the upstream termination signal enhancer of SV40 (2SV-USE) and is introduced into the U3-region of the 3'-LTR of the proviral plasmid. Because this USE-element becomes part of the U3-region of the retroviral RNA, this USE-element will be duplicated into the 5'-LTR by reverse transcription and, accordingly, will also be present in both LTRs when integrated along with the transgene into target cells.

Although presence of the USE-element according to the invention to be located within the U3-region of the 3'-LTR is preferred in vectors according to the invention, its function is independent from the novel promoter element, e.g. the RSV promoter and vice versa.

Vectors according to the invention, i.e. comprising the novel promoter element within the U3-region of the 5'-LTR, and/or the USE-element within the U3-region of the 3'-LTR, can independently further comprise a transgene intended for integration into the target cell's genome, including internal promoters and/or IRES elements functionally linked with the transgene.

Upon retroviral transduction of a target cell, both the 5' SIN-LTR and the 3'-SIN-LTR receive the poly-adenylation signal, preferably also the artificially generated 2SV element. As a result, it is to be expected that presence of the poly-adenylation signal reduces the probability for generation of fusion transcripts with adjacent cellular nucleic acid sequences, while concurrently, expression of the transgene is enhanced. In respect of fusion transcripts, read-through from the internal promoter, i.e. from the transgene expression cassette into sequences downstream within the target cell, is minimized and, due to the duplication of the polyA signal into the 5'-region, read through from upstream target cell sequences into the integrated viral sequences is concurrently minimized. Therefore, in addition to enhancing transgene expression, the polyA signal according to the invention increases biosafety of integrated artificial sequences.

For targeting of viral particles comprising the inventive vectors, viral particles may be provided with specific surface proteins, e.g. glycoprotein g of Vesicular Stomatitis Virus (VSV-G), suitable for transfection of a large variety of cells.

In a further embodiment, the novel promoter element and/or the USE-element are contained in LTR-driven retroviral vectors that contain active enhancer-promoter sequences within the U3-region. Again, such vectors may contain different transgenes, functionally linked with internal promoters and/or IRES elements to be integrated into the target cell.

In a further embodiment, the novel promoter element and/or the USE-element are contained within a retroviral vector for improved efficiency of the transfer of episomal DNA. Sequences contained within such episomes can code for various proteins, optionally functionally linked with internal promoters and/or IRES elements.

In addition to a 5 '-promoter element and a 3 '-SIN element, vectors according to the present invention comprise all additional sequence elements required to fulfil the retroviral life-cycle, such as the primer binding site (PBS), a dimerisation and packaging signal (ψ element) as well as a transgene intended for delivery into mammalian cells, arranged between the SIN-LTRs.

In a further embodiment, the novel promoter element and/or the USE-element are contained in a viral vector suitable for effective pseudo-transduction. The mRNA contained in the pseudo-transduction particles may encode different proteins, their genes functionally linked with IRES elements or proteolytic cleavage motifs.

### Detailed description of the invention

According to the invention, retroviral vectors, especially, gammaretroviral vectors, e.g. MLVs, can be constructed, as well as on the basis of foamyvirus (FV) or lentiviral vectors, e.g. based on HIV. Integration of a 5'-promoter element and/or 3'-SIN element, each according to the invention, results in vectors able to generate high titers of viral particles in permissive cells like up to 5 x 10⁷ per mL while still using self-inactivating (SIN) LTRs.

Further, it is a specific advantage of the present invention that the modification of the 3'-SIN-LTR by integration of the artificially duplicated poly-adenylation enhancer signal derived from SV40 that retroviral vectors can be obtained which can achieve high titers of retroviral particles and high efficiency of transgene expression in transduced target cells independent of the post-transcriptional regulatory element (PRE) from Woodchuck Hepatitis Virus (WPRE), which in the state of art vectors is usually positioned upstream the 3'-LTR.

Preferred usage of the vectors according to the invention is for retroviral gene transfer into somatic stem cells, especially hematopoietic stem cells, preferably of mammalian, e.g. human origin. When using the vectors according to the invention for gene therapy, they offer the advantage of minimizing unwanted activation of genes at the site of insertion into the genome.

As a first component for increasing the titer of infectious viral particles containing the gammaretroviral vector, its 5'-promoter is modified by exchanging the inherent promoter activity, which in the state of art originates from a promoter of the Myeloproliferative Sarcoma Virus (MPSV), for a promoter element that has a high transcriptional elongation rate.

At present, it is concluded from the observation that the increase in titer is only dependent on the promoter element that replaces the original P_{MPSV} in the 5'-LTR that it is possibly not the transcription initiation activity of the promoter element, but rather the transcriptional elongation rate of the promoter element which is responsible for the increase in titer. This effect of the promoter element integrated into the 5'-LTR, leading to an increase in titer can be observed for all respective internal promoters tested controlling the reporter gene or a transgene, respectively. It is assumed that in addition to the high transcriptional elongation rate of the 5'-promoter, it is its strong capability to recruit the RNA-polymerase initiation complex that results in increased transcription starting at the nucleotide +1 of the R-element, i.e. downstream of the 5'-promoter, hence producing increased titers of viral particles in permissive cells. The increased efficacy of the 5'-promoter to achieve a high transcriptional elongation rate can on the one hand be determined in relation to different promoters arranged in its place in the 5'-LTR, or on the other hand determined in relation to the efficacy of the internal promoter linked to the expression cassette of the transgene or reporter gene integrated into the viral surroundings. Accordingly, one possibility to identify suitable promoters for arrangement in the 5'-LTR of gammaretroviral constructs according to the invention is to determine the relative transcriptional and, optionally, the relative translational activities of the 5'-promoter and the internal promoter linked to a reporter gene. For the purpose of identifying a promoter having a high transcriptional elongation rate according to the invention, a dual reporter gene assay is provided, using expression of separately identifyable fluorescent reporter proteins linked to either of the 5'-promoter and the internal promoter, respectively.

Using the dual reporter gene assay, for example using reporter genes that produce distinguishable translation products, evaluation of the activities of the 5'-promoter arranged in the 5'-LTR and of the internal promoter in absolute values and in relation to each other can be obtained in a simple manner. In this assay, the relative activities of transcription products and/or translation products from the 5'-promoter and the internal promoter, respectively, indicate relative activities of these promoters, serving as a measure for transcriptional activity to drive synthesis of full-length viral RNA suitable for packaging in relation to transcripts of the transgene expression cassette. Accordingly, for the combination of promoters tested, it is possible to approximately predict the suitability of the 5'-promoter to generate high titers of viral particles.

The dual reporter gene assay is a preferred way for estimating the amount of complete viral RNA synthesized from a given viral construct and, hence the titer of viral particles to be expected in packaging cells. However, it is also possible to evaluate the amount of complete viral RNA synthesized from a viral construct comprising a certain 5'-promoter, e.g. in combination with an internal promoter by Northern blotting, wherein full-length RNA transcripts of viral sequences can easily be distinguished from the shorter RNA transcripts controlled by the internal promoter.

5'-promoters in viral constructs according to the invention having a high transcriptional elongation rate can be identified in Northern blots and, preferably, in the aforementioned dual reporter gene assay by a high level of activity, i.e. transcriptional activity in the case of Northern blotting and by expression in the case of the dual reporter gene assay. According to the invention, it is preferred that the ratio of activity of the 5'-promoter to the activity of the internal promoter is at least 1.5, preferably at least two, more preferably 5 to 10 or above.

The dual reporter gene assay uses separate structural genes, the translation products of which can be distinguished in subsequent measurements, preferably spectrophotometrically, or by fluorescence measurement, e.g. by fluorescence activated cell sorting (FACS). The dual reporter gene assay is based on the measurement of gene activities of the reporter genes, the first reporter gene under the control of the 5'-(LTR)-promoter, the second reporter gene under the control of the internal promoter, with both integrated into the viral sequence. Preferably, the first reporter gene is arranged downstream of the functional elements of viral sequences necessary for producing viral particles that contain full-length viral RNA, e.g. downstream of the 5'-promoter, the R-element, the U5-element, an optional splice donor site, and a ψ-site, but upstream of the internal promoter. Accordingly, activity of the first reporter gene indicates its effective transcription under the control of the 5'-promoter, which transcription is considered a proof for the prior transcription of the upstream sequences, i.e. of the 5' viral sequence elements. As an option for the dual reporter gene assay to test whether the 5'-promoter suppresses the internal promoter, e.g by polymerase readthrough, this first reporter gene can immediately be followed by a poly-adenylation site in 3'.

The second reporter gene forms an expression cassette with the internal promoter, which is arranged downstream of the first reporter gene, and upstream further optional viral functional elements like PRE and upstream of the 3'-LTR.

Transfection of permissive cells, e.g. packaging cells with the dual reporter gene construct comprised in a plasmid, e.g. an *E.coli* shuttle plasmid, leads to synthesis of RNA, firstly under the control of the 5'-promoter, which synthesizes full-length RNA transcripts eventually suitable for packaging into viral particles, and secondly RNA transcripts under the control of the internal promoter. It is at present presumed that transcripts initiated from the internal promoter impair the production of full-length RNA transcripts suitable for packaging into viral particles. However, transcription of the transgene under the control of the internal promoter is desired after the ultimate transduction of target cells for the expression of the transgene.

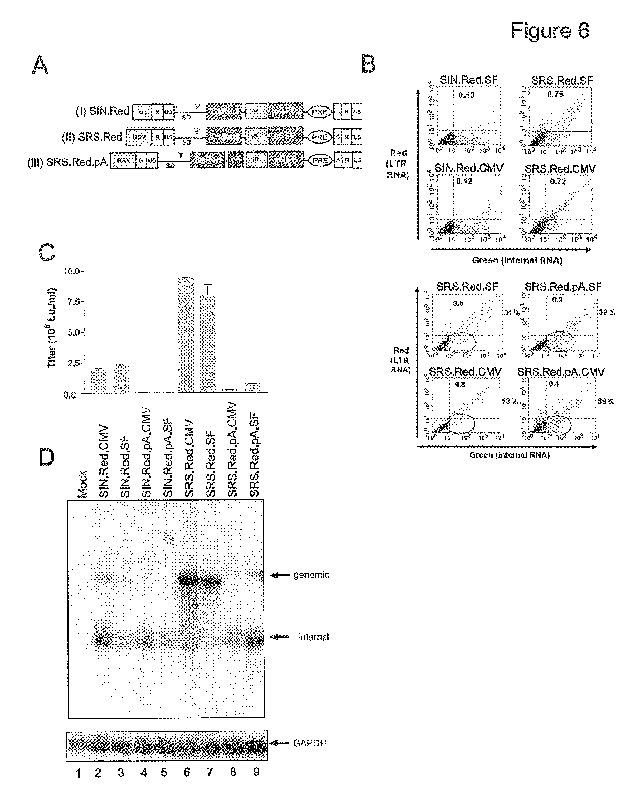

The fact that the high transcriptional activity, e.g. the high transcriptional elongation rate has been found to be an important factor for the production of high titers of viral particles from gammaretroviral vectors, which are in contrast to early generations of lentiviral vectors tat independent, implies that it is not only the basal promoter strength which is an important property of the 5'-promoter, but also its efficiency to recruit an elongation competent RNA polymerase II complex. Accordingly, the influence of the 5'-promoter on synthesis of full-length retroviral RNA, suitable for packaging, can also be derived from Figure 6 described below, wherein a modified 5'-LTR containing the 5'-promoter according to the invention (SRS), replacing the state of art 5'-promoter (P_{MPSV}), leads to an increase of viral particle titer.

Accordingly, within the context of this disclosure, the term transcriptional elongation rate is also intended to describe the property of a 5'-promoter element to increase the titer of viral particles, which is a property that has been found not to be necessarily linked with the strength of the promoter to induce transcription.

In general, both the RSV- and the tetracycline-inducible promoters have been found to lead to high SIN-vector titers, at least in gamma-retroviral vectors, and further when used in vectors on a lentiviral basis, e.g. on HIV. With specific reference to lentiviral vectors, including third generation constructs, both promoters were previously found to generate vectors suitable for tat independent production of viral particles (Dull et al., J. Virology 8463-8471 (1998), Xu et al., Mol. Ther. 97-104 (2001)). It is assumed that for both gamma-retroviral vectors and lentiviral vectors, the promoters according to the invention lead to the generation of high viral particle titers in permissive cells by recruiting an elongation competent RNA polymerase II complex. It is assumed that this elongation competent RNA polymerase II complex corresponds to the tat dependent HIV LTR, also showing that it is not simply the basal promoter strength that is the important factor for overruling an internal promoter. Accordingly, even the use of the CMV promoter, generally regarded as a stronger promoter to drive genomic retroviral RNA expression, did not give rise to higher viral titers.

As a preferred embodiment, the 5'-promoter according to the invention comprises the Rous Sarcoma Virus promoter (P_{RSV}) and, preferably, in functional arrangement with P_{RSV} the enhancer element derived from SV40, as well as the 3'-SIN-LTR comprising a USE-element derived from SV40 in the place of the deletion of the U3-region. This combination of sequences is comprised in Seq ID No. 1. In Seq ID No. 1, containing a total of 5124 base pairs, the P_{RSV} is located at bases 447 to 675, the repeat element (R) and the U5-region of the 5'-SIN is contained at bases 676 to 820, the primer binding site (PBS) is located at bases 821 to 837, the splice donor site (SD) is located at bases 882 to 883, and bases 1314 to 1714 comprise the Spleen Focus Forming virus promoter (P_{SF}), functionally linked as the internal promoter to the reporter gene, exemplified here by the gene encoding green fluorescent protein (GFP), located at bases 1776 to 2495. The 3'-SIN-LTR is located at bases 2578 to 2872, wherein a first USE-element (obtained from SV40) is located at bases 2612 to 2655, a second USE-element (duplicate of the SV40 USE-element) is located at bases 2662 to 2705, together realizing the preferred USE-element comprising an artificially duplicated USE element of SV40 (2SV), located at bases 2612 to 2705 of Seq.-ID No 1.

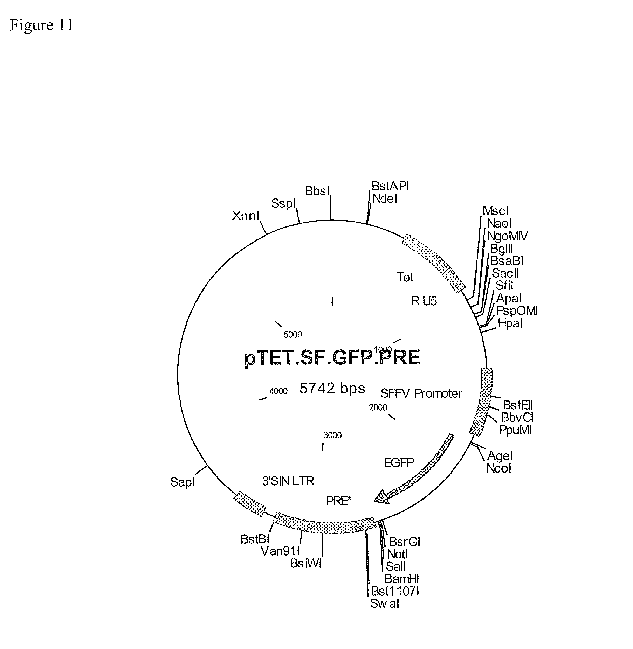

An alternative embodiment, a retroviral vector comprising the tetracyclin promoter as the 5'-promoter of the construct is given in Seq.-ID No 2, and as a schematic drawing in Figure 11.

Seq.-ID No. 2 comprises the tetracyclin inducible promoter (Tet, located at bases 447 - 762) in 5' to the R and U5 regions (located at bases 763 - 907), followed in 3' by a multiple cloning site. The 3' LTR (located at bases 3296 - 3485) also has SIN-configuration and is arranged in 3' to a PRE, namely WPRE (located at bases 2621 - 3223). The reporter gene eGFP (GFP, located at bases 1863 - 2582, the amino acid sequence is given as Seq. ID No. 3) is arranged in 5' to the WPRE and under the control of the SFFV (Spleen Focus - Forming Virus) promoter (located at bases 1401 - 1801, arranged in 5', which is an internal promoter of the U3 region of SFFV.

The invention will now be described in greater detail with reference to the figures, wherein
- Figure 1 A schematically shows the organization of gammaretroviral constructs,
- Figure 1 B shows the infectious titers obtained from packaging cells with constructs according to Figure 1 A,
- Figure 1 C shows Northern blot analyses from packaging cells with constructs according to Figure 1 A,
- Figure 2 A schematically shows gammaretroviral vectors used for the assessment of the influence of modifications in 3',
- Figure 2 B shows infectious titers obtained for vector constructs according to Figure 2 A,
- Figure 2 C shows titers obtained with vector constructs according to Figure 2 A and the mean fluorescence intensity (MFI) of transduced target cells expressing the reporter gene product eGFP,
- Figure 3 A for comparison schematically shows gammaretroviral SIN vectors, using different internal promoters,
- Figure 3 B shows the vector titers and the MFI of target cells transduced with vectors according to Figure 3 A,
- Figure 4 A for comparison schematically shows lentiviral vector constructs that comprise the inventive 5'-promoter and the inventive 3'-SIN-LTR,
- Figure 4 B shows titers and the MFI obtained with the comparative vectors according to Figure 4 A,
- Figure 5 A schematically shows gammaretroviral vectors according to the invention, modified in both their 5'- and 3'-LTRs,
- Figure 5 B shows the infectious titers obtainable for constructs according to Figure 5 A and the MFI of SC1-cells (mouse fibroblasts) transduced with the vectors according to Figure 5 A, respectively,
- Figure 6 A shows gammaretroviral vectors for comparison (I) and according to the invention (II, III) for the dual reporter gene assay,
- Figure 6 B shows the FACS results of relative expression of reporter genes from constructs of Figure 6 A in the dual reporter gene assay,
- Figure 6 C shows titers obtained for constructs of Figure 6 A,
- Figure 6 D shows a Northern blot analysis of packaging cells transfected with constructs according to Figure 6 A,
- Figure 7 A schematically shows gammaretroviral vectors for comparison (SIN) and according to the invention (SRS),
- Figure 7 B shows relative titers obtained for the vectors according to Figure 7 A,
- Figure 7 C shows FACS results of target cells transduced with viral particles containing viral constructs according to Figure 7 A,

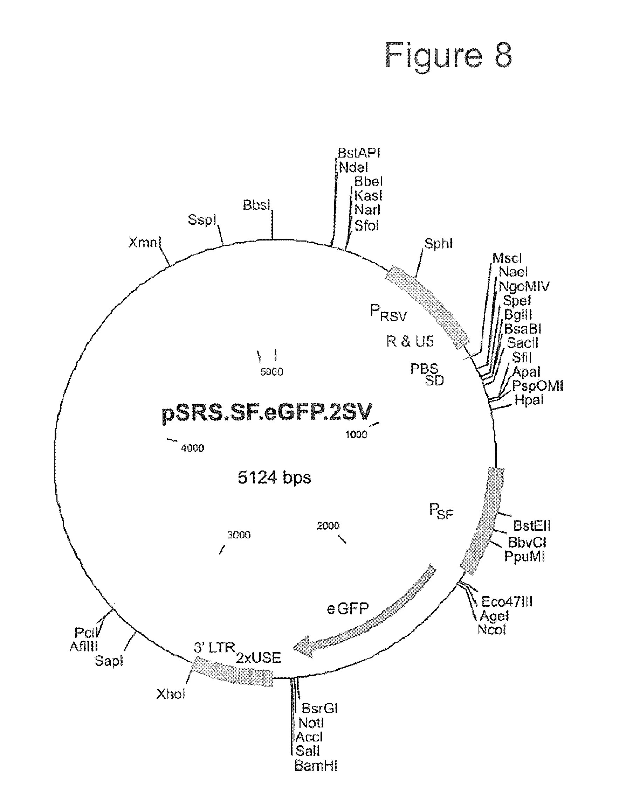

- Figure 8 represents a schematic drawing of the vector having Seq ID No. 1, and
- Figure 9 A schematically shows gammaretroviral vectors according to the invention, comprising a tetracycline-inducible promoter,
- Figure 9 B shows relative titers obtained for the vectors according to Figure 9 A,
- Figure 9 C shows Northern blotting results for packaging cells containing the vectors according to Figure 9 A,
- Figure 10 A schematically shows a vector suitable for monitoring transcriptional read-through from retroviral sequences into downstream genes of the target cell, represented by an IRES-DsRed cassette as a reporter gene,
- Figure 10 B shows graphs of reporter gene fluorescence (Y-axis) in relation to transfection efficiency (X-axis) in cells transfected with the construct of Figure 10 A, and
- Figure 11 shows a schematic drawing of a vector plasmid containing a nucleic acid construct according to the invention.

In the figures, vector constructs are depicted schematically, wherein sequences necessary for replication, maintenance and selection markers in a host bacterium, e.g. *E. coli,* are indicated by the line drawing a connection to include the viral sequences and inserts, which are represented by boxes and straight lines. The designations of exemplary vectors are assembled from their respective arrangements of functional elements, i.e. the promoter, enhancer, poly-adenylation and further elements in 5'to 3'. In these vector designations, identical elements like identical promoters or identical structural genes like reporter genes or the transgene indicated in the graphical representation may be omitted. For promoter sequences, vector designations only contain the origin of promoters, whereas the graphical schemes indicate promoters by a "P". For example, the promoter from the state-of-art 5'-SIN-LTR, namely from MPSV is indicated as P_{MPSV} and is designated in the vector simply as SIN. Similarly, the USE elements are also indicated by their origin only.

### Example 1: Identification of 5' promoter dominating potential internal promoters of 5'-SIN-LTR

When investigating ways to enhance the titer of gammaretroviral vectors suitable to be produced at high titers in permissive packaging cells, the original promoter element derived from Myeloproliferative Sarcoma Virus (P_{MPSV}) present in the state-of-art 5'-SIN-LTR (termed SIN in vector designations) was replaced by a variety of promoter elements.

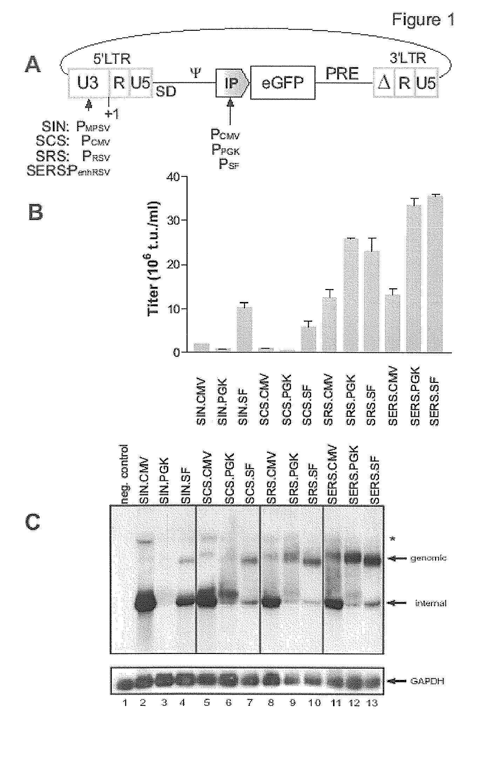

A schematic representation of said vectors is given in Figure 1 A, wherein the promoter from cytomegalovirus (P_{CMV}) replaces the MPSV promoter element (P_{MPSV}), resulting in a novel 5'-promoter termed SCS. When replacing the MPSV promoter with the promoter element from Rous Sarcoma Virus (P_{RSV}), resulting in a 5'-promoter termed SRS, and a further construct by adding enhancer sequences obtained from Simian Virus 40 (enh) to the P_{RSV}, a 5'-promoter termed SERS (enhP_{RSV}) was obtained. Each of these derivatives of the original P_{MPSV}-containing SIN element contains a 5'-promoter according to the invention, an unmodified repeat element (R), and an unmodified U5-region, followed by an optional splice donor site, and a ψ-region.

The expression cassette for a reporter gene encodes eGFP under the control of an internal promoter (IP), which was derived from cytomegalovirus (P_{CMV}), alternatively the promoter from phosphoglycerate kinase (P_{PGK}) or from Spleen Focus - Forming Virus (P_{SF}), respectively. In each construct, the reporter gene expression cassette was followed by the Woodchuck Hepatitis Virus post-transcriptional regulatory element (WPRE) and a 3'-LTR in its SIN-configuration, indicated by the delta (Δ), comprising residual U3-sequences, the R-element and a U5-region. In Figures 1 B and 1 C, vector designations SIN and SRS refer to 5 '-promoters, CMV, PGK and SF to internal promoters.

When producing viral particles from vector constructs according to Figure 1 A, which are a permutation of promoter elements present in the 5' U3-element with internal promoters linked to the reporter gene, it can be observed that the promoters according to the invention derived from Rous Sarcoma Virus (P_{RSV}), and even more so the promoter from RSV in combination with the enhancer element from SV40 (enhP_{RSV}) lead to strongly improved titers over the comparative construct comprising the P_{MPSV}. Further, this increase in titer is independent from the internal promoter coupled to the reporter gene.

It is interesting to note that it is not the promoter from CMV (P_{CMV}), which rather reduces the titers obtained, but the promoter from RSV (P_{RSV}) and enhanced P_{RSV} (enhP_{RSV}) that lead to improved titers, when considering that the latter promoters are traditionally regarded as less strong in comparison to P_{CMV}.

The results derivable from Figure 1 B are confirmed by the Northern blots shown in Figure 1 C, revealing that the 5 '-promoter elements according to the invention, namely SRS and SERS lead to the highest synthesis of genomic vector RNA. For comparative purposes, glycerol aldehyde phosphate dehydrogenase (GAPDH) was identified as an internal standard and shown in the inset.

### Example 2: Improvement of titer by modification 3'-SIN-LTR

In order to further improve the titer of retroviral vectors, the 3'-SIN-LTR is modified in its U3-region to contain an upstream signal enhancer (USE) element that promotes transcriptional termination and poly-adenylation. As a starting point for this aspect of the invention, a viral construct comprising a state-of-art 5'-LTR (P_{MPSV}) was used in combination with a state-of-art 3'-SIN-LTR, containing a major deletion within its U3-region. This deletion is known to impede retroviral transcriptional termination and poly-adenylation, resulting in a reduction of titer and a reduction of transgene expression in target cells. Further, insufficient termination increases the risk of activation of sequences downstream the insertional site, i.e. activation by readthrough and/or by splicing into cellular genes located downstream.

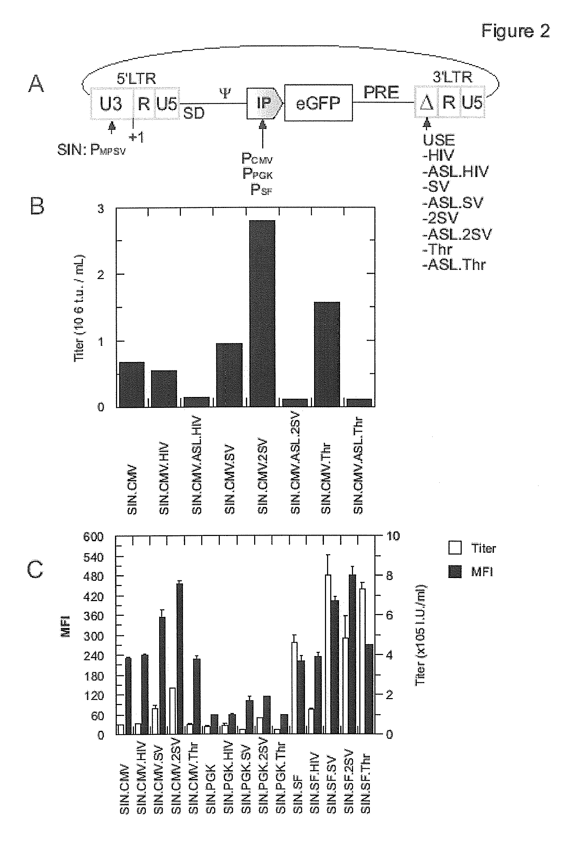

For vectors according to the invention, the USE-elements expected to promote poly-adenylation, which are derived from HIV-1 (HIV), SV40 (SV), the thrombine gene (thr), or realized by a novel, artificially duplicated SV40 element (2SV) were integrated into the deletion of the 3'-LTR. Additionally, combinations of the aforementioned USE-elements with an artificial stem-loop sequence (ASL) placed directly upstream the USE-element were created. A schematic representation of constructs is given in Figure 2 A.

Further, the internal promoter functionally linked to the reporter gene encoding eGFP was alternated between the CMV promoter (P_{CMV}), the phosphoglycerate kinase promoter (P_{PGK}) and P_{SF}.

The titers obtained in permissive cells for the constructs according to Figure 2 A are shown in Figure 2 B; Figure 2 C shows mean fluorescence intensities of the reporter gene (MFI) and titers for the SC1 target cells transduced with the retroviral vectors. In Figures 2 B and 2 C, vector designation SIN refers to the 5'-promoter; vector designations CMV, PGK and SF refer to internal promoters, and HIV, SV, 2SV, and Thr refer to USE elements. The results shown in Figures 2 B and 2 C demonstrate that highest titers and best transgene expression could be achieved for the duplicated SV40 USE-element (2SV) with no artificial stem-loop sequence, and further demonstrates that this USE-element enhances titers and transgene expression independent from the internal promoters tested for the reporter gene.

This example shows that both titer in permissive cells and transgene expression in target cells can be enhanced by inserting a USE-element into the site of deletion of the 3' U3-region of a 3'-SIN-LTR. Best activity in each respect was found by the artificially duplicated USE-element derivable from SV40, termed 2SV. Note that expression in target cells was studied under experimental conditions that use an identical multiplicity of infection for all vectors.

The modification of the 3'-SIN-LTR has the advantage of resulting in the production of high viral titers in a large variety of permissive cells, i.e. production of a high titer is essentially independent from the cell type.

### Example 3: Influence of promoter controlling transgene expression and a PRE

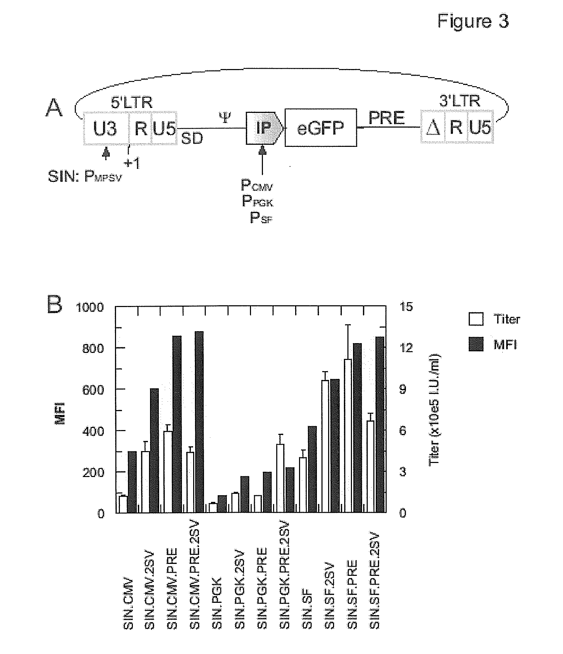

For examination whether the effect the preferred USE-element, namely 2SV, is influenced by the internal promoter controlling the expression of the transgene contained in the vector or whether it is influenced by a PRE-element, variations of a gammaretroviral vector having a conventional 5'-LTR comprising a U3-region having an MPSV promoter, but a 3'-SIN-LTR comprising the USE-element 2SV in the place of the deletion of the 3' U3-region, was used for variation of the internal promoter controlling the reporter gene eGFP. Further variations were the presence of the PRE-element upstream of the 3'-SIN-LTR. The vectors are schematically represented in Figure 3 A.

In Figure 3 B, vector designation SIN refers to the 5'-promoter; vector designations CMV, PGK and SF refer to internal promoters. 2SV and PRE refer to the USE element and the post-transcriptional regulatory element of Woodchuck hepatitis virus, respectively.
The titers obtained in permissive cells and the MFI obtained in retrovirally transduced SC 1 target cells are depicted in Figure 3 B, showing that the USE-element 2SV increases both titer of viral particles as well as expression of the transgene, essentially independent of the internal promoter controlling the reporter gene as well as independent of the PRE element.

### Comparative Example 1: Lentiviral vector

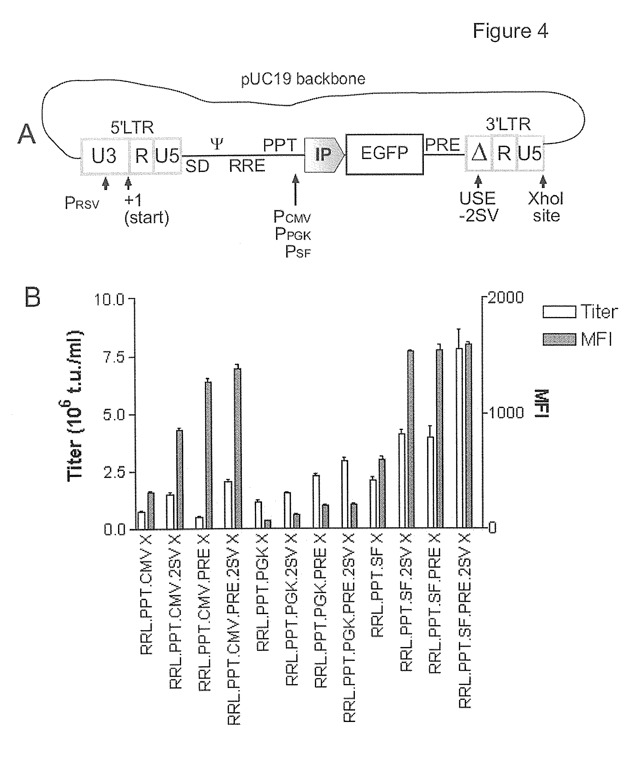

In order to examine whether a 3'-SIN-LTR containing the preferred USE-element (2SV) in the place of the deletion of the U3-region can also elevate titer and expression of state-of-the-art lentiviral vectors derived from HIV-1, its 3'-SIN-LTR was replaced by this element according to the invention. The reporter gene (eGFP) was put under the control of internal promoters CMV, PGK or SF. These comparative lentiviral vectors are schematically depicted in Figure 4 A.

In Figure 4 B, vector designations indicate the 5'-promoter P_{RSV} contained in all constructs. Vector designations PPT refer to the identically contained PPT element, whereas vector designations CMV, PGK and SF refer to internal promoters, and 2SV, RRE refer to content of the USE element and Rev responsive element, respectively, wherein PRE refers to the WPRE element. X refers to a modification of the plasmid sequences located downstream the 3'LTR obtained by cloning the lentiviral vector into a pUC 19 backbone that lacked a polyadenylation signal of its own. The USE positioned in the 3'U3 region interacts better with the polyadenylation signal located in the R region of the lentiviral 3'LTR.

The resulting titers in permissive cells and the MFI of retrovirally transduced SC1 target cells, i.e. the transgene expression, are shown in Figure 4 B. These results shown in Figure 4 B indicate that both titer and transgene expression are increased by presence of the preferred USE-element 2SV in the 3'-SIN-LTR of lentiviral vectors. To achieve this, an element of the lentiviral vector plasmid located in 3'of the 3'LTR had to be removed because it was found to interfere with the introduction of the USE-element.

The results also show that the USE-element is effective independent of the WPRE.

### Example 4: Combined modification of the 5'-promoter and 3'-SIN-LTR according to the invention

In order to examine a possible synergistic effect of the 5'-promoter having a promoter of high transcriptional elongation rate according to the invention by combination with a 3'-SIN-LTR according to the invention, comprising the 2SV USE-element in the place of the deletion of the U3-region, gammaretroviral vectors were constructed for comparison and according to the invention.

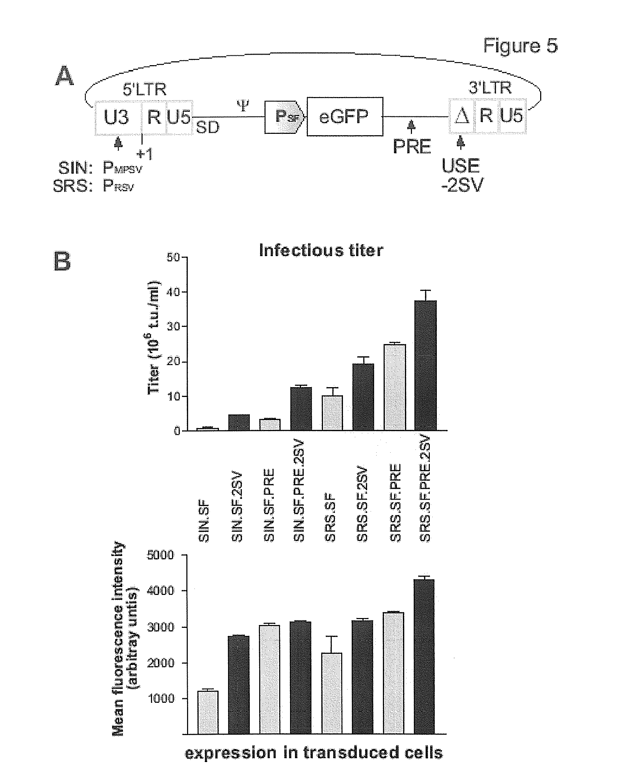

Schematically, the vectors are shown in Figure 5, wherein SIN indicates the cloning site of the 5' U3-region, containing the state of art promoter element from Myeloproliferative Sarcoma Virus (P_{MPSV}) for comparative purposes. In vectors according to the invention, the P_{MPSV} was replaced by the Rous Sarcoma Virus promoter (P_{RSV}). In Figures 5 B and 5 C, vector designations SIN and SRS refer to 5'-promoters P_{MPSV} and P_{RSV}, repectively, whereas SF indicates P_{SF} as the internal promoter. PRE and 2SV indicate presence of the PRE and USE element, respectively.

For more detailed functional analysis, a PRE-element could be introduced between the reporter gene expression cassette (SF controlling the eGFP gene) and the 3'-SIN-LTR. Further, the 3'-SIN-LTR having the deletion of the U3-region could be replaced by a 3'-SIN-LTR carrying a USE-element according to the invention, represented by the preferred USE-element 2SV in the place of the deletion of the U3-region which is found in state of art vectors.

Results obtained for vectors according to Figure 5 A in respect of the infectious titer are shown in Figure 5 B, including transgene expression as measured as MFI in retrovirally transduced SC1 target cells. As shown in Figure 5 B, both titer and expression level are enhanced by concurrent presence of both the 5'-promoter and the 3'-SIN-LTR according to the invention in a synergistic manner to higher values than resulting from each element according to the invention, respectively.

### Example 5: Determination of activities of the 5'-promoter and of the internal promoter by Northern blotting and the dual reporter gene assay

Gammaretroviral constructs for the determination of the ratio of transcription activities under the control of the 5'-promoter and under the control of the internal promoter, respectively, reporter gene sequences were integrated into different gammaretroviral vectors as depicted in Figure 6 A, showing the gammaretroviral region of the plasmids only, namely
I) a comparative state of art gamma-retroviral vector (SIN.Red) containing the MPSV promoter,
II) a vector according to the invention, termed SRS.Red, containing the RSV promoter in its 5'-LTR, and,
III) a vector according to the invention termed SRS.Red.pA, comprising a poly-adenylation signal (pA) in 3' to the first reporter gene.

In these vectors, a first reporter gene is the cDNA for the dsRed Express fluorescent protein (Bevis et al., Nature Biotechnology 20, 83-87 (2002)), arranged directly upstream the internal promoter, the latter controlling the gene for eGFP (enhanced green fluorescent protein) that serves as the second reporter gene.

In Figures 6 A to D, vector designations SIN and SRS refer to 5'-promoters P_{MPSV} and P_{RSV}, repectively, whereas SF indicates P_{SF} as the internal promoter. The second reporter gene representing the transgene in all the constructs measured, is not indicated in vector designations. In vector designations, CMV, SF indicate internal promoters P_{CMV} and P_{SF}, respectively, whereas pA indicates presence of the poly-adenylation signal to the first reporter gene dsRed Express.

For the dual reporter gene assay using distinguishable fluorescent reporter genes, e.g. eGFP and dsRed Express, 2 µg transfer vector, 10 µg M57-DAW (encoding MLV gag-pol), 2 µg ecotropic MLV env were transfected into Phoenix-gp cells using the calcium phosphate technique. Three days after infection, packaging cells were analysed by FACS. Compensation of FL-1 (eGFP) and FL- 2 (dsRed express) was performed using monofluorescent constructs.

A marker gate was set at the mean fluorescence intensities for eGFP, and dsRed-Express positive cells were calculated accordingly.

The translational activity driven from each of the 5'-promoter and the internal promoter, respectively, was analysed in transfected Phoenix-gp cells. The results from FACS analysis measuring both green fluorescence, caused by expression of dsRed Express and green fluorescence, caused by expression of eGFP, are depicted in Figure 6 B, wherein the graphs show the ratio of red fluorescence to green fluorescence signals for the indicated constructs.

The FACS results show that SIN vectors having the conventional MSPV promoter in their 5'-LTR show an unfavourable ratio of green (second reporter gene expression) versus red fluorescence (first reporter gene expression), which is shown as the quotient of the Y versus X mean fluorescence intensity (Figure 6 B, left panel). In contrast, modification of the 5'-LTR according to the invention by introducing the RSV promoter as an example for a promoter having increased translational elongation efficiency, increased this ratio about 6-fold. Dot-plot analysis revealed that the effect of the RSV promoter was even independent of the expression level (Figure 6 B, right hand panels).

Vector constructs comprising the poly-adenylation signal (in this case derived from bovine growth hormone) downstream the first reporter gene further increased the ratio of mean fluorescence intensities (Figure 6 B, central panel) of second reporter gene expression versus first reporter gene expression, which is also reflected in a drastic decrease in titers (Figure 6 C).

In Figure 6 B, the right circle in the middle panel marks cells that intensify their green fluorescence after insertion of the poly-adenylation signal. On the right-hand side beneath the dot plots, transfection efficiencies of this particular experiment are given.

These results confirm the applicability of the dual reporter gene assay for a determination of suitable promoter elements for the 5'-LTR, and for a determination of suitable combinations of 5'-promoters with internal promoters for gammaretroviral vector constructs, because it confirms the assumption that synthesis of full-length retroviral RNA from the 5'-promoter is essential for obtaining high titers of viral particles (Figure 6 C).

For Northern blots, total RNA was prepared and Northern blot analysis was performed as known in the art. In general, 10 µg RNA isolated from transfected cells was separated at 0.6 V/cm² for 5 hours in denaturing formaldehyde gels. Subsequently, RNA was transferred to membrane (0.45 µm Biodyne-B, Pall Corporation, Pensacola, USA) by capillary transfer and fixed for 2 hours by heating to 80 °C. Hybridization was done according to standard procedures. As probe, 100 ng corresponding to the PRE fragment, present in the vector constructs, was radiolabelled to an activity of the least 10⁶ cpm/mL hybridization solution and separated from non-incorporated labelled nucleotides on spin columns. After hybridization, filters were washed and exposed to X-ray film and quantified by phospoimaging.

Northern blot analyses, shown in Figure 6 D, support the dual reporter gene assay in that highest relative (and absolute) levels of transcription of full-length viral RNA, i.e. under the control of the 5'-promoter, were found for the gammaretroviral constructs according to the invention, namely SRS11.Red.CMV and SRS11.Red.SF. Determinations of the titers obtained in this experiment are shown in Figure 6 C, indicating highest titers for gammaretroviral constructs according to the invention.

Designations arranged in between Figures 6 C and 6 D are valid for both these figures to identify vector constructs.

Further, these results show that the insertion of the first reporter gene further increased the titer obtained for the construct according to the invention having the RSV promoter in the 5'-LTR, in relation to the same gammaretroviral construct which only differs in not containing the first reporter gene expression cassette. This increase in titer is presently interpreted as a spacer effect, i.e. the increase in titer of retroviral particles by increasing the number of nucleotides between the 5'-LTR and the internal promoter driving the expression cassette's transcription.

### Example 6: Retroviral transduction of primary hematopoietic cells

As an example for target cells to be genetically manipulated by transduction with gammaretroviral vectors according to the invention, murine primary hematopoietic cells were transduced with gammaretroviral vectors containing the MPSV promoter (P_{MPSV}) as the 5'-promoter for comparative purposes (designated SIN in the vector) and the RSV promoter (P_{RSV}) as the 5'-promoter according to the invention (designated SRS in the vector). The viral sequence elements are schematically shown in Figure 6 A, wherein P_{SF} indicates the spleen focus forming virus promoter serving as the internal promoter driving transgene expression.

Further, transgene expression cassettes containing the spleen focus forming promoter functionally linked to the MGMT gene and to the eGFP gene, respectively, followed by the PRE-element from Woodchuck Hepatitis virus, were compared for viral particle titers obtained in packaging cells, and for transgene expression in transduced target cells.

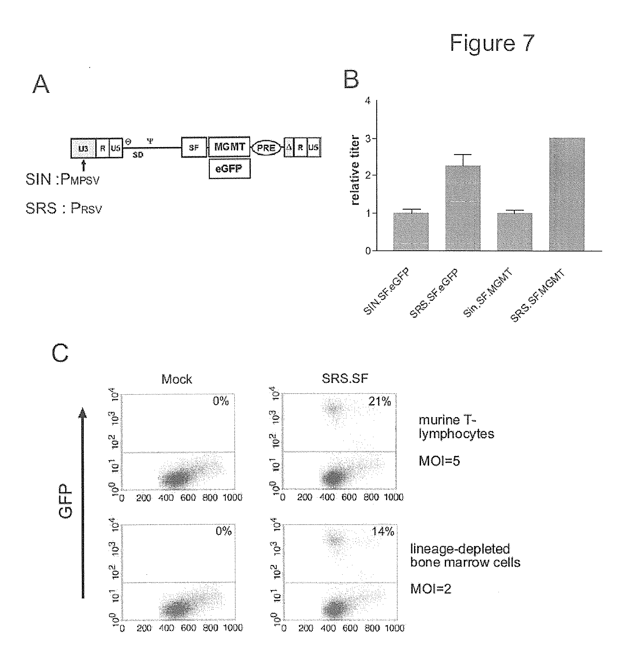

In Figures 7 B and 7 C, vector designations SIN and SRS refer to 5'-promoters P_{MPSV} and P_{RSV}, respectively. Reporter genes whereas SF indicates P_{SF} as the internal promoter. eGFP and MGMT indicate structural genes contained as the transgene.

Viral titers were determined in SC1 packaging cells expressing the retroviral vector constructs. The titers obtained are shown in Figure 7 B in relation to the titer obtained for the conventional vector termed SIN. SF (titer taken as 1, 5'-promoter is P_{MPSV} and internal promoter is P_{SF}), demonstrating a strong increase in titer for the vector constructs according to the invention, essentially independent of the transgene.

The results for the transgene expression in transduced murine primary hematopoietic cells four days after transduction with the vectors according to Figure 7 A is shown in Figure 7 C. For transduction, an MOI of 5 was used for SRS.SF.eGFP supernatants (upper panels), and an MOI of 2 for SRS.SF.eGFP transduction in lineage-depleted bone marrow cells in (lower panels).

### Example 7: Increase of vector titer by using a tetracycline-inducible promoter

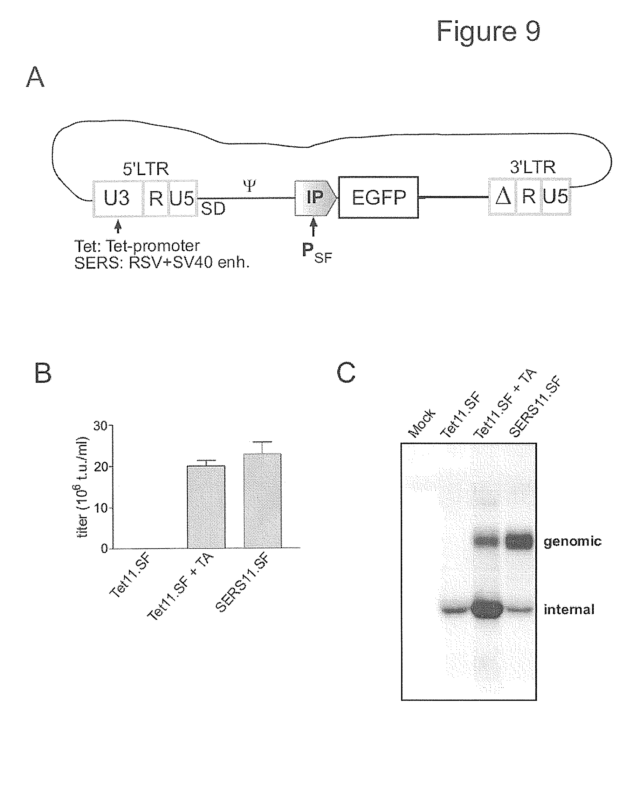

As an alternative promoter to the one obtainable from Rous Sarcoma Virus (P_{RSV}) used in Example 1, the tetracycline-inducible promoter was introduced into the U3-region of the 5'-LTR. Both vector constructs are schematically shown in Figure 9A, showing as I (corresponding to the construct SRS of Figure 1 using P_{SF}) a construct using the P_{RSV} (RSV) and the Pₜₑₜ (TRE). As a reporter gene, eGFP was included under its own SF promoter, followed by WPRE.

For cloning a tetracycline-inducible promoter into the 5'-LTR for driving full-length vector RNA synthesis, the promoter fragment was cloned from plasmid ptES₁-1(g)p (obtainable from Dr. Rainer Löw, EUFETS AG, Idar-Oberstein, Germany) by PCR using primers 5'Tet11afl (Seq. ID No 4: 5'-GCTACTTAAGCTTCTTT CACTTTTCTCTGTCA-3') and 3'Rkpn (Seq. ID No 5: 5'-GAGAACACGGGTACCCGG GC-3'). The tetracycline-inducible promoter consists of a tet operator hexamer with 4 C specificity, fused to the Moloney murine leukemia virus (MMLV) minimal promoter. The resulting PCR fragment of 315bp was cloned into the *Afl*II and *Kpn*I sites of the 5'-LTR of the pSRS 11.SF construct, shown in Figure 9A under I. All PCR fragments were confirmed by DNA sequencing.

For vector production of the tetracycline-inducible vector, 5 µg of the expression plasmid, harbouring the authentic trans-activator with 4 C DNA binding specificity was co-transfected into 5x10⁶ Phoenix-gp or 293 T-cells as permissive cells.

The titers are shown in Figure 9B, indicating titers of viral particles obtained in permissive cells of about 2x10⁷ transducing units /mL supernatant when transfection was done with transactivator plasmis (TA, 5 µg), whereas the titer was significantly lower when transfection was done without transactivator (TA). The construct containing the Rous Sarcoma Virus promoter (SERS11.SF) yielded even high titers.

In Figure 9C, the corresponding Northern blot analysis of the packaging cell line (Phoenix-gp) is shown. Interestingly, the amount of internal transcript is also increased for the tetracycline promoter containing construct, which could be interpreted as an interaction between the 5' end and the internal promoter. However, the construct containing the RSV promoter and the SV40 enhancer still yielded a higher concentration of genomic RNA (Figure 9C, lane 4), however, this increased RNA concentration did not translate into higher titers. At present, it is assumed that the limiting factor are available resources of Gag/Pol or Env, as inferred from experiments using saturating plasmid amounts.

In summary the present results show that the tetracycline-inducible promoter is useful for high titer production of gammaretroviral SIN vectors.

### Example 8: The prevention of transcriptional readthrough by the USE - modification of the 3'-SIN-LTR 4

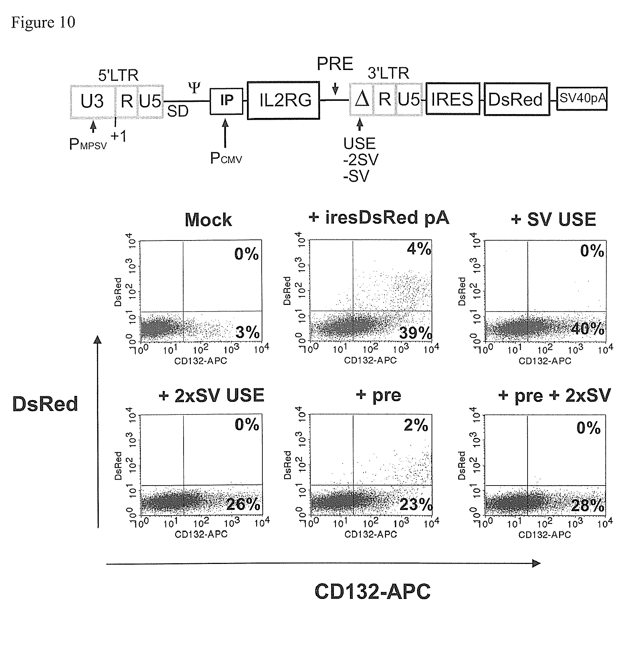

In order to monitor the potential transcriptional readthrough from the retroviral promoter over the poly-adenylation and termination motives located in the 3'-SIN-LTR into a downstream gene of the target cell genome, a reporter plasmid was generated, which in addition to the retroviral vector components contains the following cassette: Downstream of the 3'-LTR an internal ribosomal entry site (IRES), derived from encephalomyocarditis virus, followed by a fluorescence marker gene (dsRed) and a poly-adenylation (pA) motif derived from SV40. The IRES ensures that the dsRed mRNA can be translated even if another open reading frame is located upstream the potential fusion transcript that may be generated by read-through over the 3' SIN-LTR. The USE-elements derived from SV40 (SV) or its dublicated form (2SV, in Figure 10 as 2xSV) were introduced into the ΔU3-region of the 3'-LTR. In some constructs, the WPRE sequence was introduced upstream of the ΔU3-region, alternatively with or without a USE. The retroviral construct according to the invention, followed by a downstream reporter gene having its own IRES sequence is schematically shown in Figure 10 A.

293 T-cells were transfected with equal amounts of the reporter plasmid and cells were analysed by flow cytometry two days after transfection.

The transfection efficiency was determined using a monoclonal antibody (anti-CD 132- APC), directed against the interleukin-2 receptor gamma-chain (IL-2), which was expressed under the control of the internal CMV promoter of the retroviral vector.

In Figure 10B, dot-plots are depicted with the fluorescence of dsRed protein (DsRed) on the Y-axis plotted over the transfection efficiency on the X-axis, as determined by CD132-APC labelling.

These results demonstrate that without addition of the USE, about 10% of transfected cells express dsRed (4% in the upper right quadrant compared to 39% in the lower right quadrant), indicating transcriptional readthrough. Addition of the SV or the 2SV-USE is sufficient to effectively block expression of dsRed.

However, WPRE (+pre) alone does not suppress transcriptional readthrough (8.6%, i.e. 2% of a total of 23% transfected cells), but WPRE can be combined with the 2SV without compromising its termination enhancer function. These results were confirmed by Northern blot analysis of total cellular RNA (not shown).

## Claims

1. Retroviral vector having a 5' LTR and a 3'-SIN-LTR, **characterized in that** the U3-region or the deletion of the U3-region of the 3'-SIN-LTR is replaced by a poly-adenylation enhancer element.

2. Vector according to claim 1, **characterized in that** the poly-adenylation enhancer element is derivable from Simian Virus 40 (SV).

3. Retroviral vector according to claim 1 or 2, **characterized in that** the poly-adenylation enhancer element is formed by an artificially created direct repeat of a poly-adenylation enhancer element obtainable from Simian Virus 40 (2SV).

4. Retroviral vector according to one of the preceding claims, **characterized in that** the U3-region of the 5' LTR comprises a promoter element having a high transcriptional elongation rate.

5. Retroviral vector according to claim 4, **characterized in that** the promoter element having a high transcriptional elongation rate is a promoter obtainable from Rous Sarcoma Virus (P_{RSV}) or a tetracycline-inducible promoter.

6. Retroviral vector according claim 4 or 5, **characterized in that** the promoter element having a high transcriptional elongation rate is preceded in 5' by promoter enhancer elements (enh).

7. Retroviral vector according to one of claims 4 to 6, **characterized in that** the promoter enhancer element is derivable from Simian Virus 40.

8. Retroviral vector according to one of the preceding claims, **characterized in that** the vector is a gammaretroviral or a lentiviral vector.

9. Retroviral vector according to one of claims 4 to 8, **characterized in that** the promoter element having a high transcriptional elongation rate corresponds to or hybridizes to the sequence defined by nucleotides -203 to -1 of Seq ID No. 1, and the poly-adenylation enhancer element corresponds to or hybridizes to the sequence defined by nucleotides 2612-2655 of Seq ID No. 1 or by nucleotides 2612 to 2705 of Seq ID No. 1.

10. Retroviral vector according to one of claims 4 to 9, **characterized in that** the promoter element having a high transcriptional elongation rate corresponds to or hybridizes to the sequence defined by nucleotides 447 to 762 of Seq ID No. 2.

11. Retroviral vector according to one of claims 4 to 10, **characterized in that** its 5' SIN-LTR corresponds to or hybridizes to the sequence defined by nucleotides 447 to 830 of Seq ID No. 1 and its 3'-SIN-LTR corresponds to or hybridizes to the sequence defined by nucleotides 2578 to 2872 of Seq ID No. 1.

12. Retroviral vector according to one of claims 9 to 11, **characterized in that** hybridization is under non-stringent or stringent conditions.

13. Viral particle, **characterized in that** it comprises a vector according to one of the preceding claims.

14. Use of a viral particle according to claim 13, for the production of a pharmaceutical composition for gene therapy.

15. Use according to claim 14, **characterized in that** gene therapy is used to transduce mammalian cells *in vivo* or *in vitro,* preferably hematopoietic stem cells or lymphocytes.
